(19) Europäisches Patentamt
European Patent Office
Office européen des brevets

(11) Publication number: 0 279 742 A2

(12) **EUROPEAN PATENT APPLICATION**

(21) Application number: 88400328.6

(22) Date of filing: 15.02.88

(51) Int. Cl.⁴: C 07 B 37/10
C 07 C 69/753

(30) Priority: 17.02.87 CA 529934

(43) Date of publication of application: 24.08.88 Bulletin 88/34

(84) Designated Contracting States: CH DE FR GB IT LI

(71) Applicant: Université de Sherbrooke
Boulevard de l'Université
Sherbrooke Québec, J1K 2R1 (CA)

(72) Inventor: Deslongchamps, Pierre
R.R. 1, Chemin McFarland
North Hatley Quebec J0B 2C0 (CA)

(74) Representative: Mongrédien, André et al
c/o SOCIETE DE PROTECTION DES INVENTIONS 25, rue de Ponthieu
F-75008 Paris (FR)

(54) **Method for the synthesis of tricyclic compounds.**

(57) The present invention relates to a novel process in which a dienophile having the following general formula (XIV):

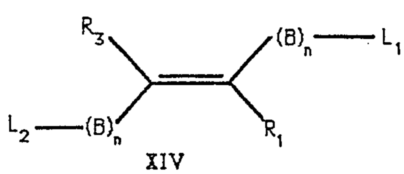

XIV

is reacted with a diene having the following formula (XV):

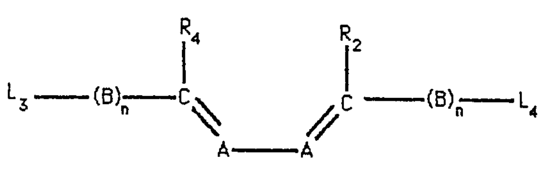

XV

in the presence of a connector of the formula:

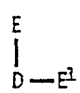

in order to afford tricyclic compounds having the following general formula (I):

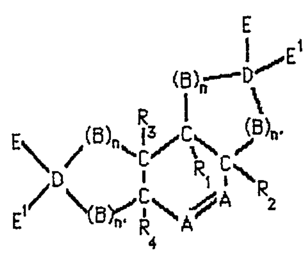

(I).

EP 0 279 742 A2

**Description**

METHOD FOR THE SYNTHESIS OF TRICYCLIC COMPOUNDS

**Background of the invention**

Progress in organic synthesis is being achieved through the development of new reactions, new experimental conditions and new strategies. The discovery of new synthetic strategies is especially important for the construction of complex organic molecules. With this thought in mind,simple intramolecular process such as cyclization are now recognized as very powerful synthetic routes.

It has been recently speculated that transannular reactions on macrocycles could very well be a much more powerful strategy than simple cyclization of aliphatic chains and provide more accurate results on a selectivity viewpoint.

However, this approach has so far been neglected because simple and direct general methods to produce macrocyles in acceptable yields were not available. Also, indirect methods to produce macrocycles such as ring expansion are not appealing because of their complexity and lack of generality. It should also be pointed out that until recently, complex macrocycles were difficult to analyze from the point of view of conformation and structure.

As a result, the synthesis of large carbocyclic and heterocyclic molecules is an area where little progress has been made. In fact, although the progress made by organic chemists in the last 50 years, from both theoretical and experimental standpoints, can be considered as exceptional, the study of conformationally restricted large molecules has almost been neglected.

In recent years, when attempts were made to synthesize products containing a macrocyclic ring, the formation of the large ring was always left for the end of the synthesis, hoping that it would be closed without too much difficulty. In other words, the large ring was rarely part of the synthetic strategy.

As mentioned earlier, direct macrocyclization of aliphatic chains presents major drawbacks. In fact, the synthesis of medium and large ring systems by such a method is disfavored mainly by both the entropy effect, that is the frequency with which atoms placed at the end of a chain will come into reacting distance, and the enthalpy effect which is the sum of steric and stereoelectronic interactions due to ring closure.

One interesting solution has been suggested to attempt circumvention of these problems, namely the replacement of methylene groups by double bonds in order to diminish the degrees of freedom of the chains as well as transannular steric interactions in the course of cyclization. This approach has demonstrated very promising perspectives.

Furthermore, now that powerful purification and analytical tools are available, it has become as easy to purify and analyse medium- and large-ring compounds as it is for aliphatic chains.

As a result, the use of macrocyclic products and transannular processes for the synthesis of polycyclic natural compounds, such as steroids, is now possible.

Therefore, a simple and direct method providing new synthetic strategies for the construction of complex organic molecules such as polycyclic molecules would be highly desirable.

**Summary of the invention**

In accordance with the present invention, there is provided a new and improved process for the synthesis of polycyclic molecules of natural and non-natural origins.

The compounds which can be prepared in accordance with the method of the present invention correspond to the following general formula (I):

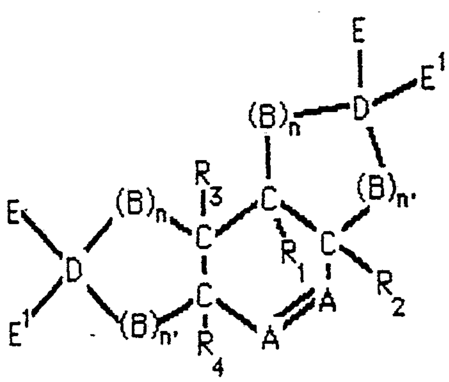

(I)

wherein:

$R_1$, $R_2$, $R_3$ and $R_4$ can be the same or different and are hydrogen, lower alkyl, aryl, aldehyde, -SR, -OR or -COOR wherein R is hydrogen, lower alkyl, or lower aryl; $-CONR_5R_6$ wherein $R_5$ and $R_6$ can be the same or

different and can be a hydrogen, lower alkyl or lower aryl; $-NR_5R_6$ wherein $R_5$ and $R_6$ are as previously defined;
A can be either nitrogen or a carbon atom, which may be substituted by $R_1$,
B can be either oxygen, sulfur, nitrogen or carbon which may be substituted by $R_1$,
C can be either nitrogen or a carbon atom, which may be substituted by $R_1$,
D can be either oxygen, nitrogen or a carbon atom, which may be substituted by E and $E^1$,
n and n′ can be the same or different and are integers ranging from 0 to 2 and
E and $E^1$ can be the same or different and may be hydrogen or a functional group.

**Detailed description of the invention**

The compounds of the present invention are thus prepared by attaching a diene and a dienophile in a convergent manner using functional groups which can remain part of the resulting molecule. The reaction may be specifically illustrated in the following manner:

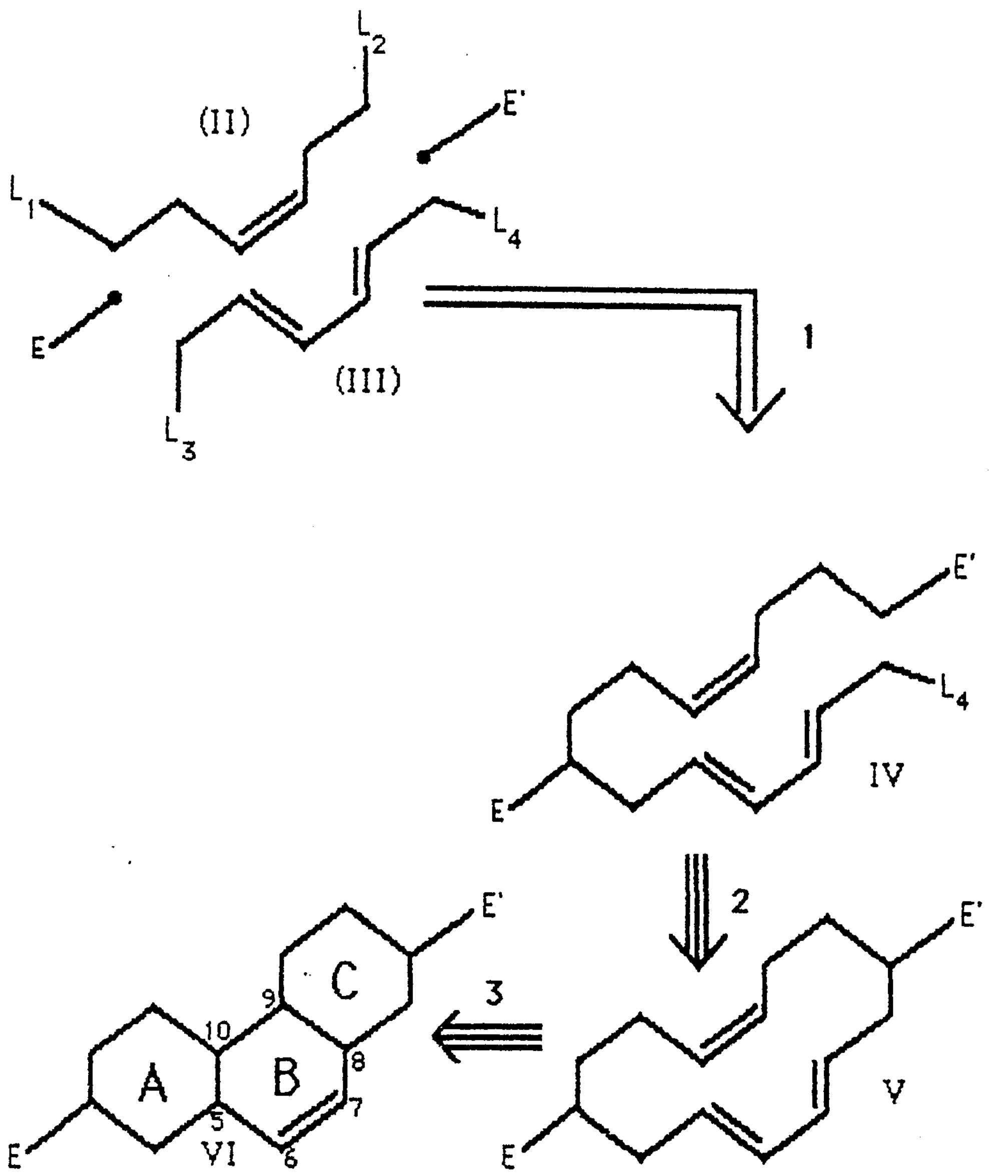

Thus, an ABC tricyclic system VI possessing chiral centers at the C-5, C-8, C-9 and C-10 positions (steroid numbering) and one or more functional groups in each ring for further elaboration can be easily produced. One can readily appreciate that this method is simple, highly convergent and contains a minimum number of steps. Furthermore, very good control is obtained in terms of reactivity, regiochemistry and relative and absolute stereochemistry.

In steps **1** and **2**, connectors E and E′ react with both diene III and dienophile II on specific leaving group sites, $L_1$, $L_2$, $L_3$ and $L_4$ in order to form an intermediate IV which is cyclized to macrocycle V. Then, in step **3** the newly formed macrocycle V undergoes transannular Diels-Alder reaction in order to form distinct cycles and since both the diene and the dienophile are part of macrocycle V, the reaction occurs without the necessity of having an activated diene and/or dienophile. Since in this case the diene and the dienophile can approach each

other in only one regioselective manner, the often difficult problem of regioselectivity is thus automatically resolved.

From a stereochemical point of view, a diene can have four different configurations (trans-trans, trans-cis, cis-trans and cis-cis) while a dienophile can have two (trans or cis). This means that the macrocyclic triene can have 8 different configurations but it can be asserted that only 7 of the 8 geometrical isomers will undergo transannular Diels-Alder reaction.

Molecular model analysis shows that from these 7 isomers, 5 macrocyclic trienes will each lead to one racemic tricyclic diastereoisomer, while the other 2 remaining macrocyclic trienes will each lead up to two racemictrcyclic diastereoisomers. The eighth macrocyclic triene (diene cis-cis and dienophile trans) is not expected to undergo a transannular Diels-Alder reaction unless there is a geometrical isomerization of one double bond during the process.

These structural limitations result from the fact that the Diels-Alder reaction has to take place via a boat transition state. Thus, the following tricyclic stereochemistries can be obtained via the method of the present invention.

| Diene geometry | Dienophile geometry | Triene geometry | Tricycle stereochemistry |
|---|---|---|---|
| trans cis | trans | trans cis trans | cis anti cis |
| cis trans | trans | cis trans trans | cis anti cis |
| trans cis | cis | trans cis cis | cis syn trans |
| cis trans | cis | cis trans cis | trans syn cis |
| cis cis | cis | cis cis cis | cis syn cis |
| trans trans | trans | trans trans trans | trans anti cis and/or cis anti trans |
| trans trans | cis | trans trans cis | trans syn trans and/or cis syn cis |
| cis cis | trans | cis cis trans | nil |

In cases where two diastereoisomers can be produced, variations in substituents E and E′ can lead to the obtention of only one of the two diastereoisomers. The use of one chiral substituent attached to the macrocyclic triene is expected to induce the preferred formation of one tricyclic enantiomer. Therefore, the production of optically active tricycles having a given absolute configuration can easily be envisaged

Furthermore, this novel method of the present invention can serve as a basis for the synthesis of natural di- or tri- terpenes and their corresponding non-natural stereo isomers. Since these compounds are polycyclic systems which contain more than 3 rings in most cases, many synthetic routes can be selected.

Therefore, if it is desired to prepare a pentacyclic molecule having, for example, the following structure:

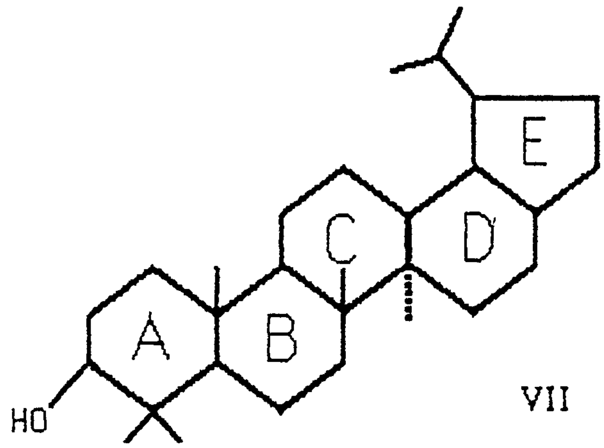

VII

it will then be possible to synthesize this molecule via three different tricyclic intermediates. Thus, either tricyclic molecule ABC(VIII), BCD(IX) or CDE(X) (shown below) could be used as a valuable starting material.

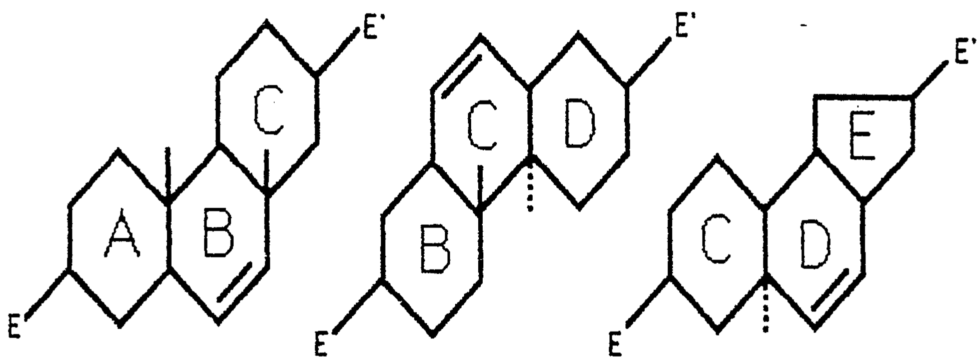

VIII IX X

and the desired terpene VII could be obtained by the different selections of each of the functional groups E and E′ in each tricyclic molecule VIII, IX or X.

One limitation concerning the three above mentioned tricycles is that the middle cycle will always be limited to a six-membered ring because of the transannular Diels-Alder reaction. There is, however, no specific limitation as to the size of the other two rings forming the tricycle.

It is also very important to stress the fact that ring substituents can be introduced by incorporation on the diene and/or dienophile, thereby avoiding the very often difficult task of introducing them later in a subsequent step.

In the case of a tetracyclic molecule such as the following steroid XI:

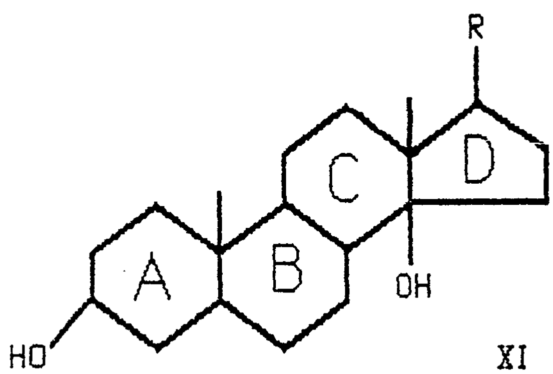

XI

at least two different tricyclic intermediates XII or XIII could be used for its preparation:

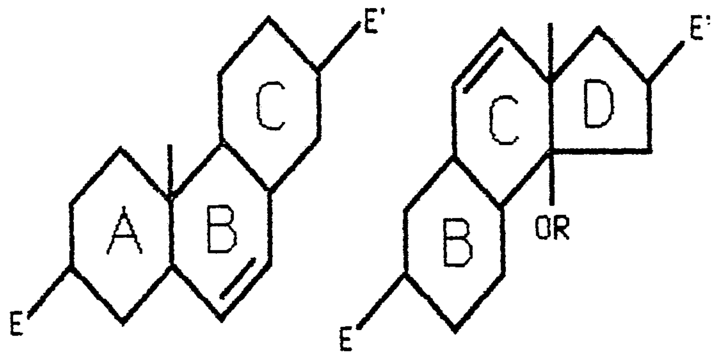

XII XIII

It is therefore possible to foresee the synthesis of polycyclic molecules containing four or five rings in the same manner the tricyclic intermediates of the present invention are synthesized.

In summary, the synthetic process of the present invention provides for easy preparation of tricyclic compounds with the possibility of introducing desired substituents prior to cyclization, thus producing a variety of molecules that can either be used *per se* or as key intermediates in the synthesis of a large number of natural or non-natural polycyclic products.

More specifically, the process of the present invention can be used for the preparation of compounds having the following general formula (I):

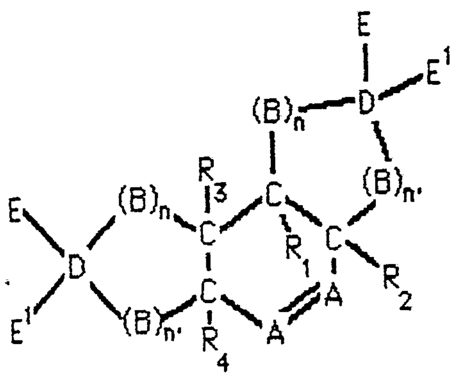

(I)

wherein:

$R_1$, $R_2$, $R_3$ and $R_4$ can be the same or different and are hydrogen, halogen, lower alkyl, aryl, aldehyde, -SR, -OR or -COOR wherein R is hydrogen halogen, lower alkyl, or lower aryl; $-CONR_5R_6$ wherein $R_5$ and $R_6$ can be the same or different and can be a hydrogen, lower alkyl or lower aryl; $NR_5R_6$ wherein $R_5$ and $R_6$ are as previously defined;

A can be either nitrogen or a carbon atom, which may be substituted by $R_1$,

B can be either oxygen, sulfur, nitrogen or carbon which may be substituted by $R_1$,

C can be either nitrogen or a carbon atom, which may be substituted by $R_1$,

D can be either oxygen, nitrogen or a carbon atom, which may be substituted by E and $E^1$,

n and n′ can be the same or different and are integers ranging from 0 to 2 and

E and $E^1$ can be the same or different and may be hydrogen or a functional group.

The synthesis process of the present invention thus comprises reacting a dienophile having the following general formula XIV:

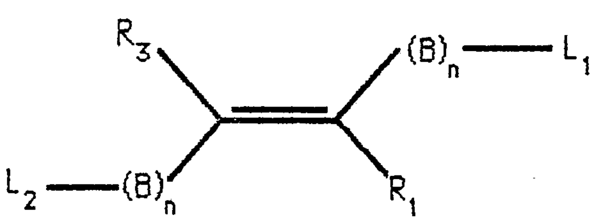

XIV

wherein

$L_1$ and $L_2$ can be the same or different and are suitable leaving groups,

B, n, $R_1$ and $R_3$ are as previously defined,

and a diene XV having the following general formula:

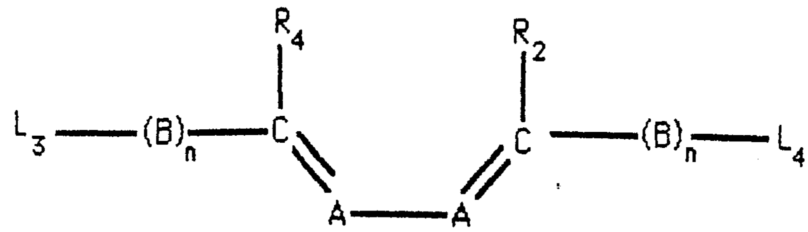

XV

wherein

$L_3$ and $L_4$ can be the same or different and are suitable leaving groups, and

B, A, C, $R_4$, $R_2$ and n are as previously defined, In the presence of a connector having the following formula:

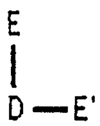

wherein D is oxygen, nitrogen or carbon and E and E′ can be the same or different and are as previously defined.

The macrocyclic-triene thus formed can subsequently undergo a transannular Diels-Alder reaction to yield the desired tricyclic compound of formula (I).

A general outline of the process of the present invention will be readily understood by referring to the following reaction scheme:

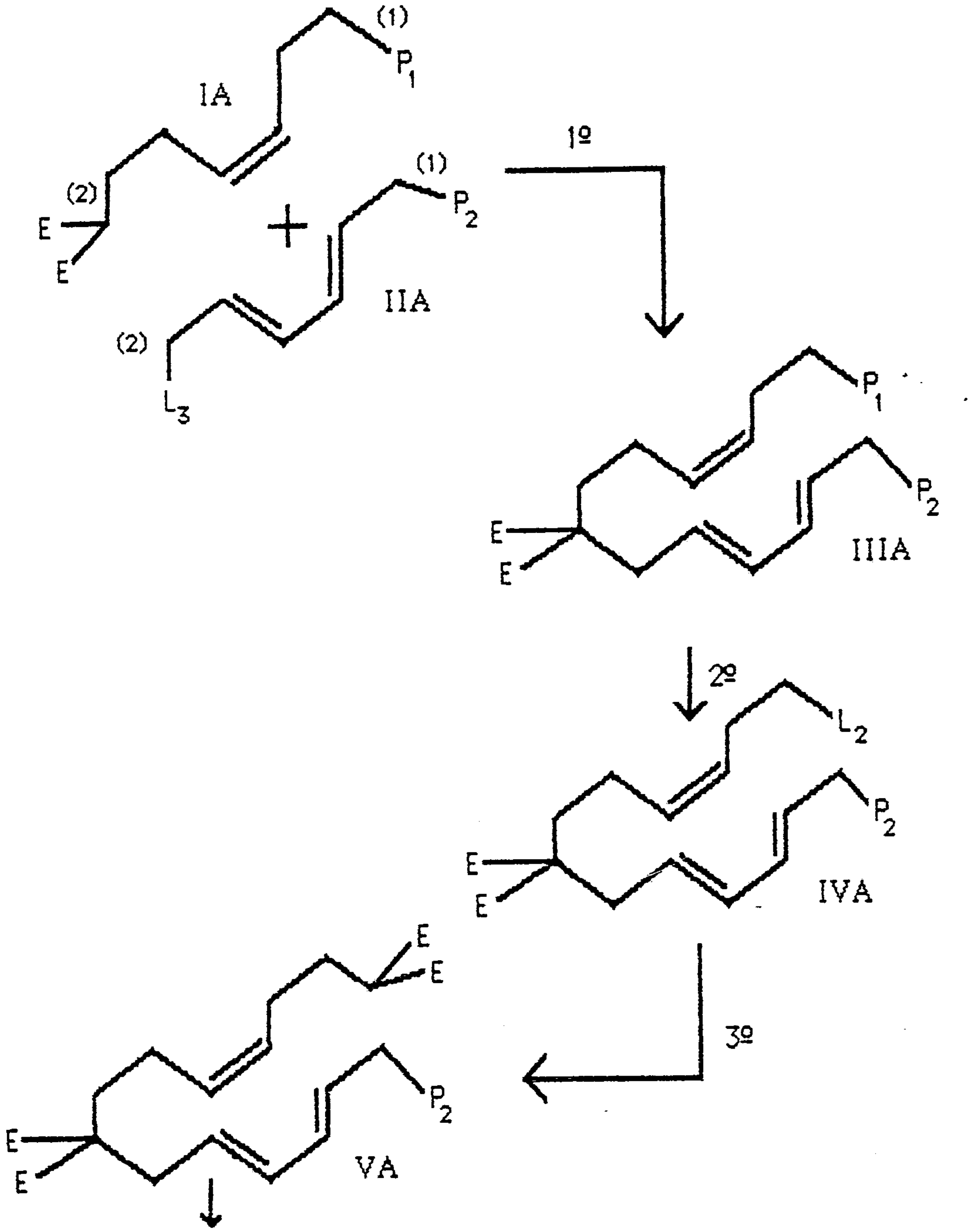
IA
IIA
1º
IIIA
2º
IVA
3º
VA

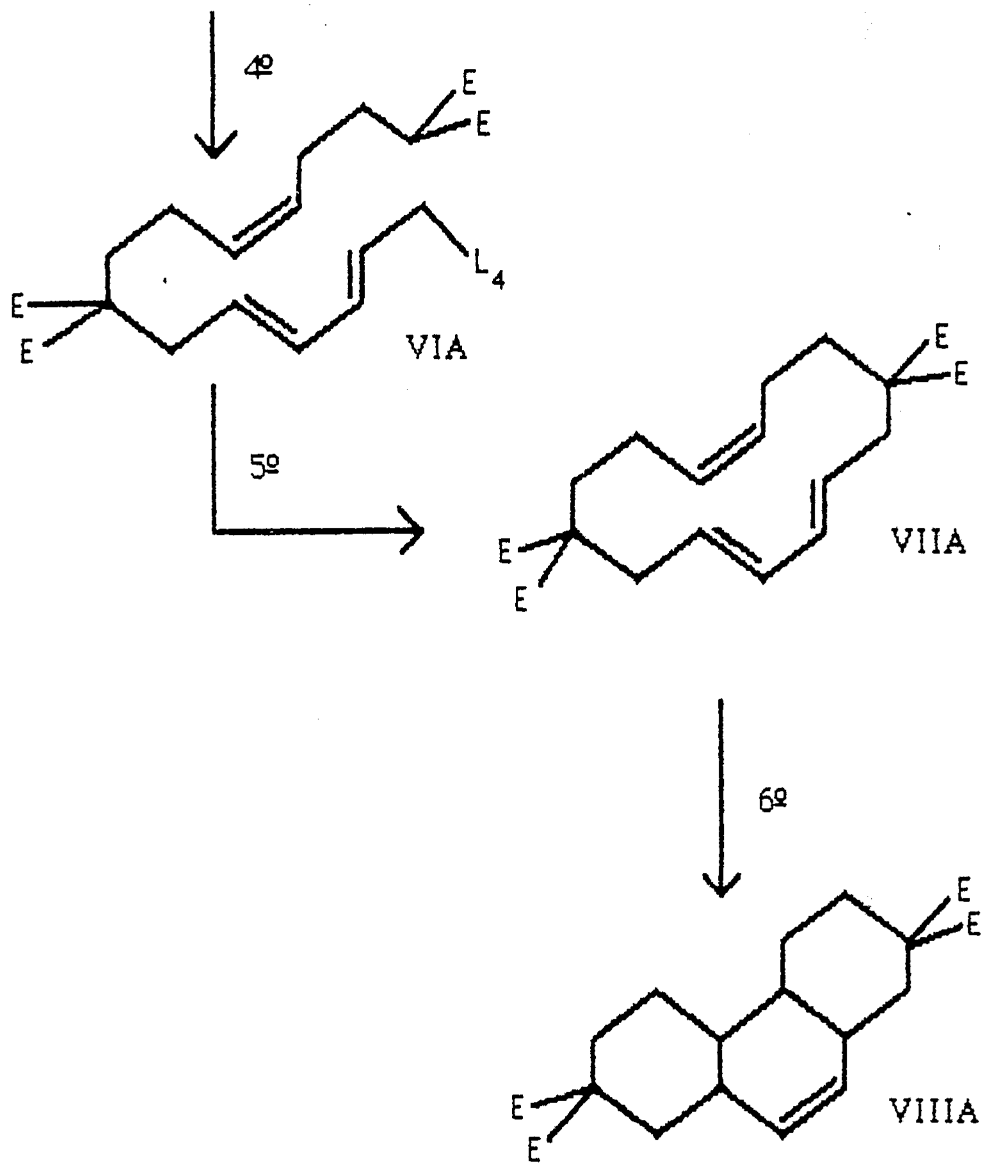


VIA

VIIA

VIIIA

Describing now a general outline of the process of the present invention, a dienophile IA having one of its leaving group sites alternatively protected by a group $P_1$ such as a silyl ether, a tetrahydropyranyl ether, a methoxymethyl ether, an ethoxymethyl ether or a lower alkyl or aryl ester, this protecting group being selected preferably from silyl or tetrahydropyranyl ether or from lower alkyl or aryl ester, more preferably, the protecting group being *tert*-butyldimethylsilyl ether, and alternatively having at one of its ends (2) either one connecting group which may be selected from the following: malonate ester, malononitrile, cyanohydrin ether or ester, 1,3-dithiolane or 1,3-dithiane, dithioacetal, sulfoxyde, sulfone, nitro or any suitable of the connecting groups described by Hase and Koskimies in Aldrichimica Acta 14, 73 (1981) and in Aldrichimica Acta 15, (2), 328 (1982) preferably from the following: dimethyl malonate or 1,3-dithiane acetal, and more preferably from dimethyl malonate, or a suitable leaving group, is reacted with a diene IIA also having one of its leaving group sites (1) alternatively protected by a group selected from the protecting groups mentioned above but preferably from silyl or tetrahydropyranyl ether or from alkyl or aryl ester, and even more preferably, the diene protecting group being a *tert*-butyldiphenyl silyl ether and alternatively having at its end (2) either a leaving group $L_3$ (shown) if the dienophile molecule bears a connecting group at its end (2) (shown) or a connecting group as defined above if the dienophile molecule bears a leaving group at its end (2), in a solvent which can be selected from tetrahydrofurane(THF), 1,2-dimethoxy ethane(DME) or dimethyl formamide(DMF), preferably from a 1:1 mixture of DMF and THF, at a temperature ranging from 25 to 90ºC, preferably at 25ºC.

The triene IIIA thus obtained is then treated in step 2º with a suitable conventional reagent (e.g. pyridinium tosylate in methanol or isopropanol, potassium carbonate or sodium carbonate in methanol, tetrabutyl ammonium fluoride in THF or hydrochloric acid in acetone, this agent being preferably selected from pyridinium tosylate in isopropanol or potassium carbonate in methanol) in order to remove one of the protecting groups that may have been introduced earlier on the dienophile IA (shown) and/or the diene IIA and

to introduce a leaving group $L_2$ (shown on dienophile) which can be selected from halide, mesylate and tosylate on the site left vacant by the removal of the protecting group. The removal of the protecting group and the introduction of the leaving group are accomplished in a conventional manner. In the context of the present invention, mesylate and chloride represent the preferred leaving groups.

The resulting compound IVA is then alternatively treated with a solution containing a connector, which can also be introduced on the dienophile IA and/or diene IIA prior to the first reaction between the dienophile IA and the diene IIA, said connector being selected from the connectors mentioned earlier, more specifically, the connector will be selected from malonate ester or dithioacetal, and even more specifically from dimethyl malonate, the reaction temperature being set, preferably between 25 and 80uC.

In a further step 4u, the remaining protecting group $P_2$ is removed from the resulting triene VA by reacting the latter with a suitable conventional; reagent. That protecting group $P_2$ may have been introduced earlier on the dienophile IA and/or the diene IIA (shown). The leaving group $L_4$ replacing the protecting group $P_2$ which can be selected from halide, mesylate or tosylate. The removal reaction is carried out as previously disclosed and leaving group introduction is accomplished in conventional manners. In the context of the present invention, mesylate and chloride would represent the preferred leaving groups.

The new leaving group site $L_4$ of triene VIA will then react with the connector previously introduced on the dienophile IA (shown) or the diene IIA in the presence of a reacting base which may be selected from potassium or sodium hydride if the leaving group is on the dienophile or sodium carbonate, potassium carbonate, cesium carbonate or any equivalent thereof, if the leaving group is on the diene, cesium carbonate being the preferred reacting base in that case (shown). This reaction is carried out in a solvent which may be selected from tetrahydrofurane (THF), 1,2-dimethoxyethane(DME) or dimethylformamide(DMF), preferably from a 1:1 mixture of DMF and THF. As for temperature, the reaction may be performed between 50 and 90uC, preferably between 80 and 85uC. Macrocyclic compound VIIA is thus afforded as shown in step 5u.

This macrocyclic compound VIIA is then dissolved in a solvent selected from aliphatic or aromatic hydrocarbons such as benzene, toluene or xylene although the reaction can sometimes be carried out without the use of any solvent, and heated at a temperature ranging from 25 to 400 uC, preferably between 75 and 350 uC, and even more specifically at 300 uC for a period of time varying between 30 minutes and 6 hours in order to afford the desired tricyclic structure VIIIA.

Furthermore, an alternative route leading to the synthesis of a triene having a structure similar to that of compound VIIA is described in the following reaction sequence illustrated by flow diagram A, wherein R, $R_1$, $R_2$ ,$R_3$ and $R_4$ are as previously defined, and where the use of connectors E to favor the attachment of diene 2a to dienophile precursor 1a is demonstrated.

## Flow diagram A

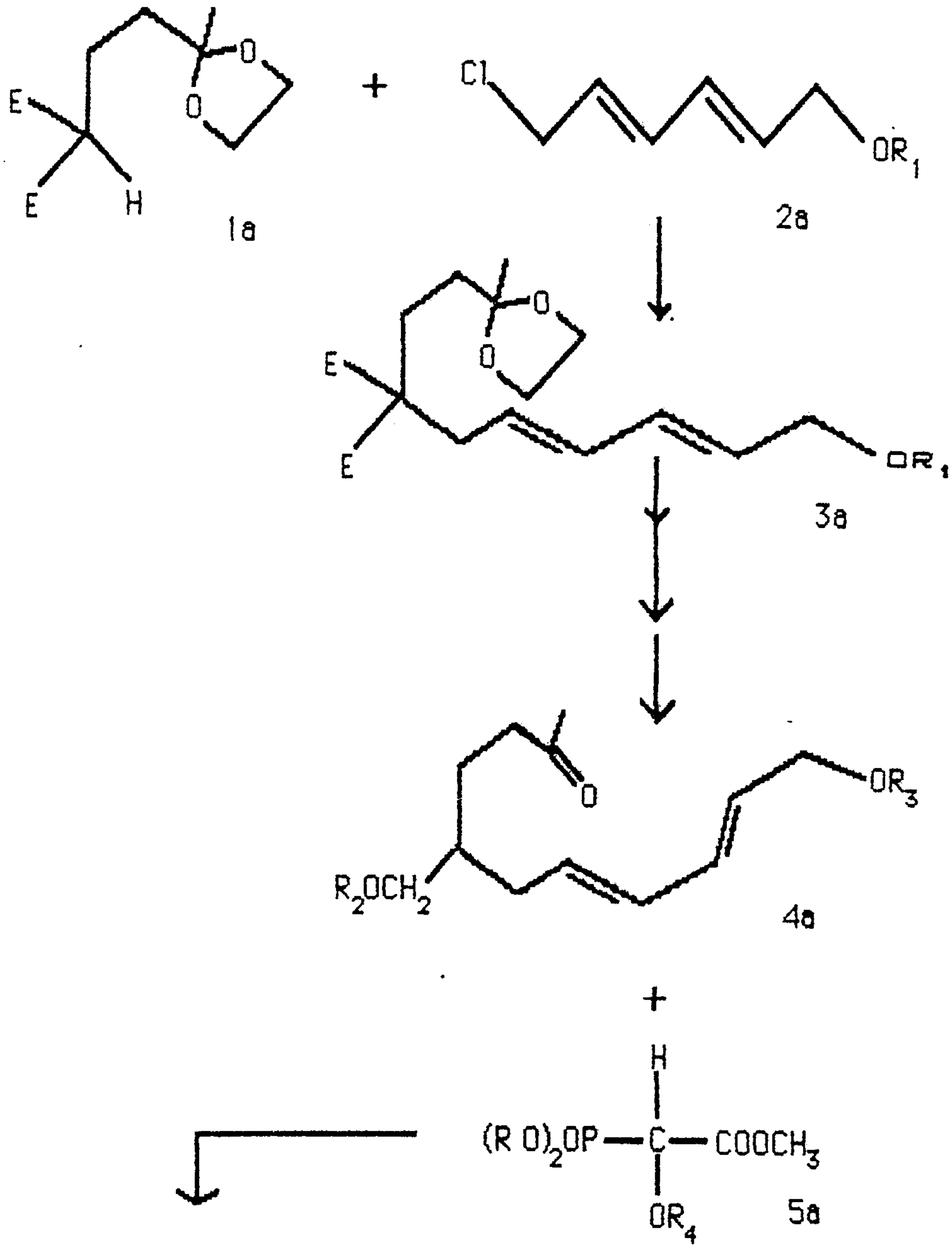

1a 2a 3a 4a 5a

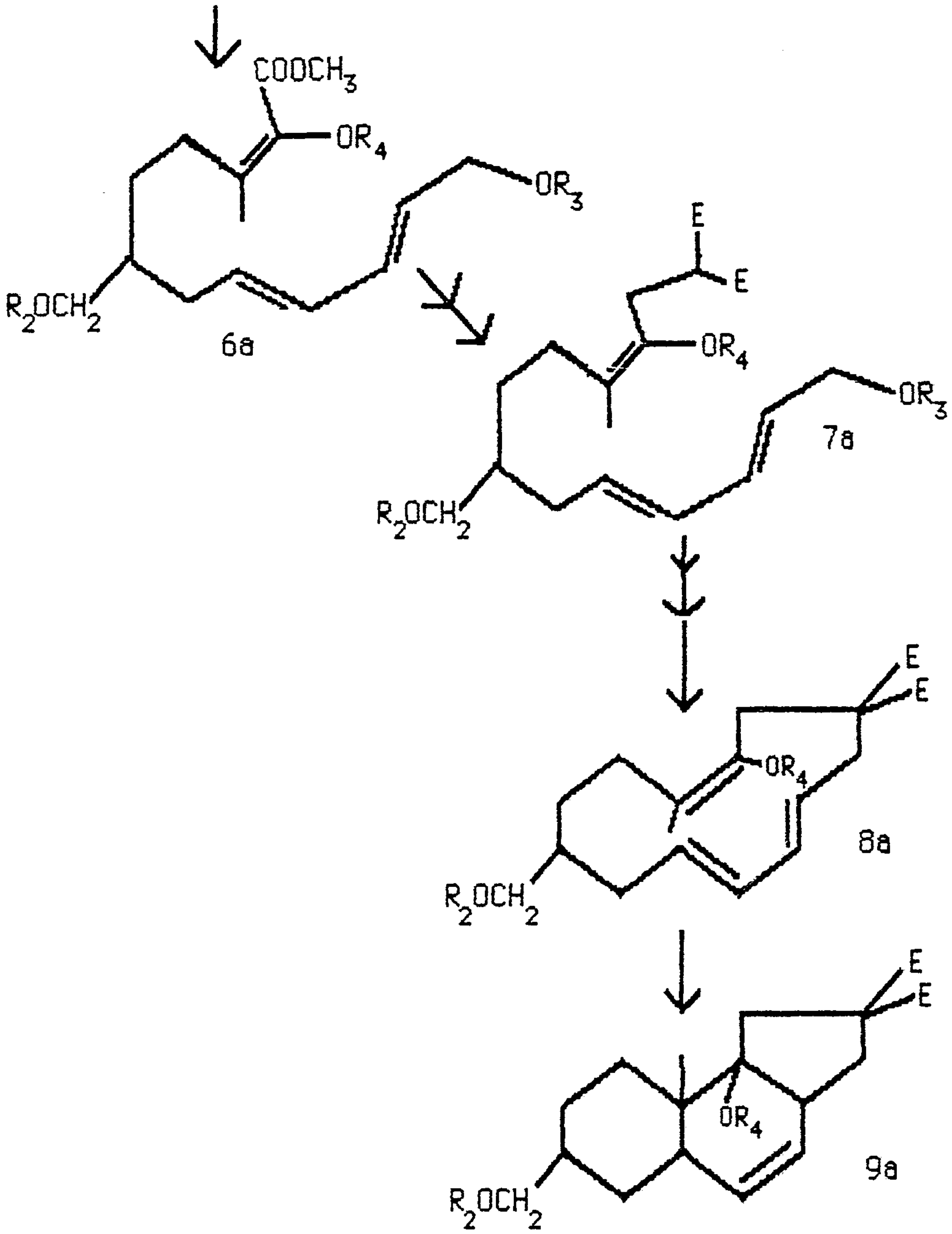

6a

7a

8a

9a

Analogs of macrocycle VIIA could alternatively be synthesized via the reaction sequences illustrated by flow diagrams B and C, wherein $R_1$, $R_2$, $R_3$ and $R_4$ are as previously defined and X is halogen. In diagram B, compound 8b is cyclized to produce macrocyclic diene 9b which is then transformed into macrocyclic triene 10b from which tricyclic compound 11b can be obtained.

## Flow diagram B

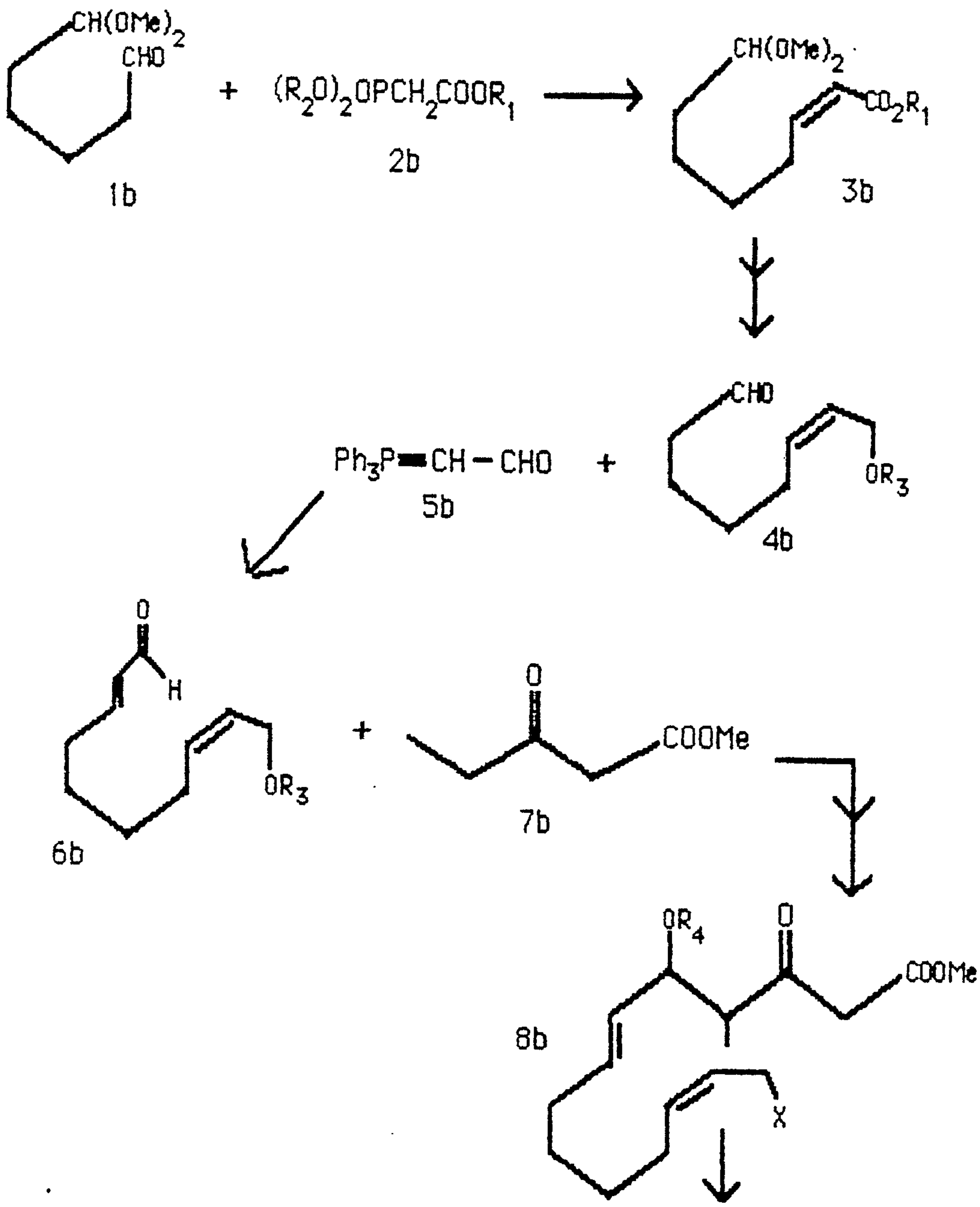

1b 2b 3b 4b 5b 6b 7b 8b

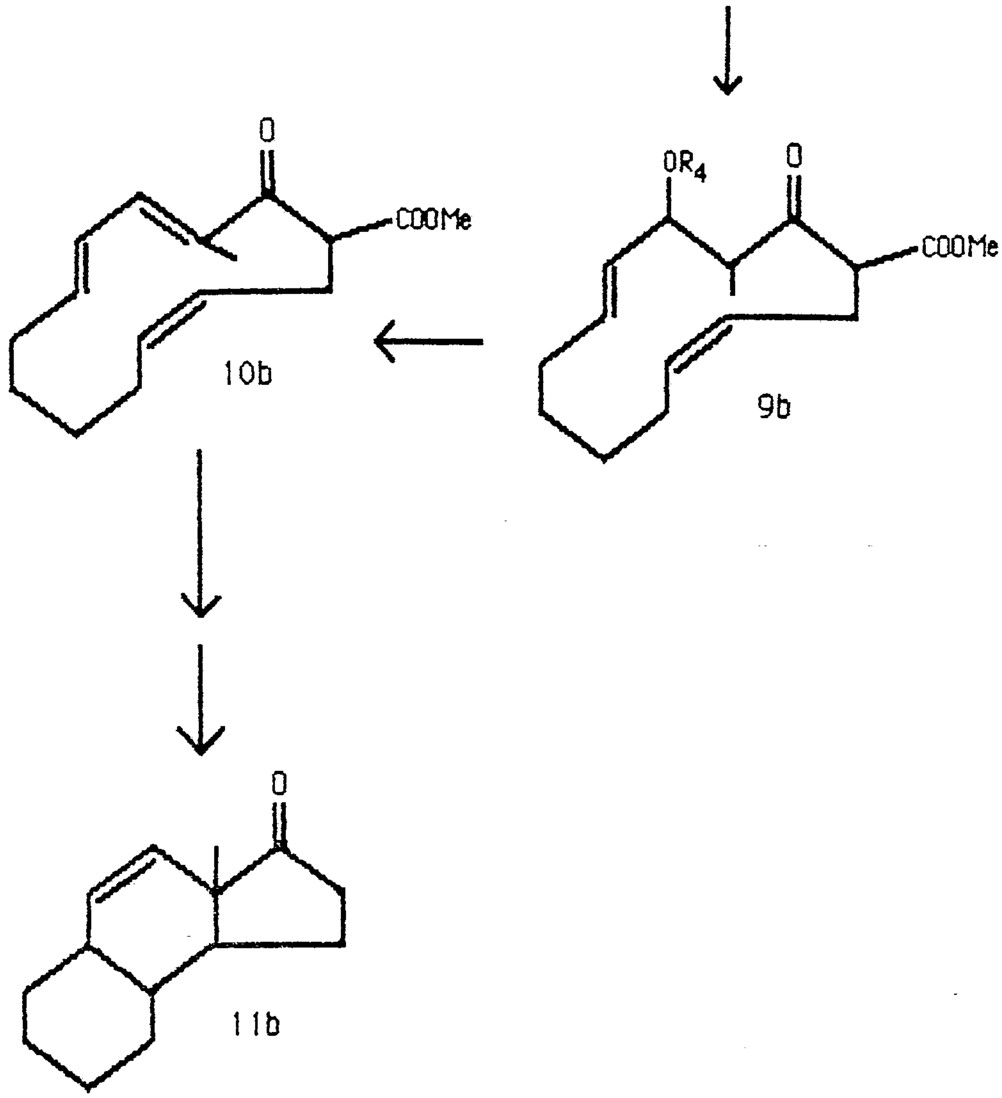

10b

9b

11b

Similarly, in diagram C, tricyclic compound 10c can be obtained from acyclic precursor 7c.

## Flow diagram C

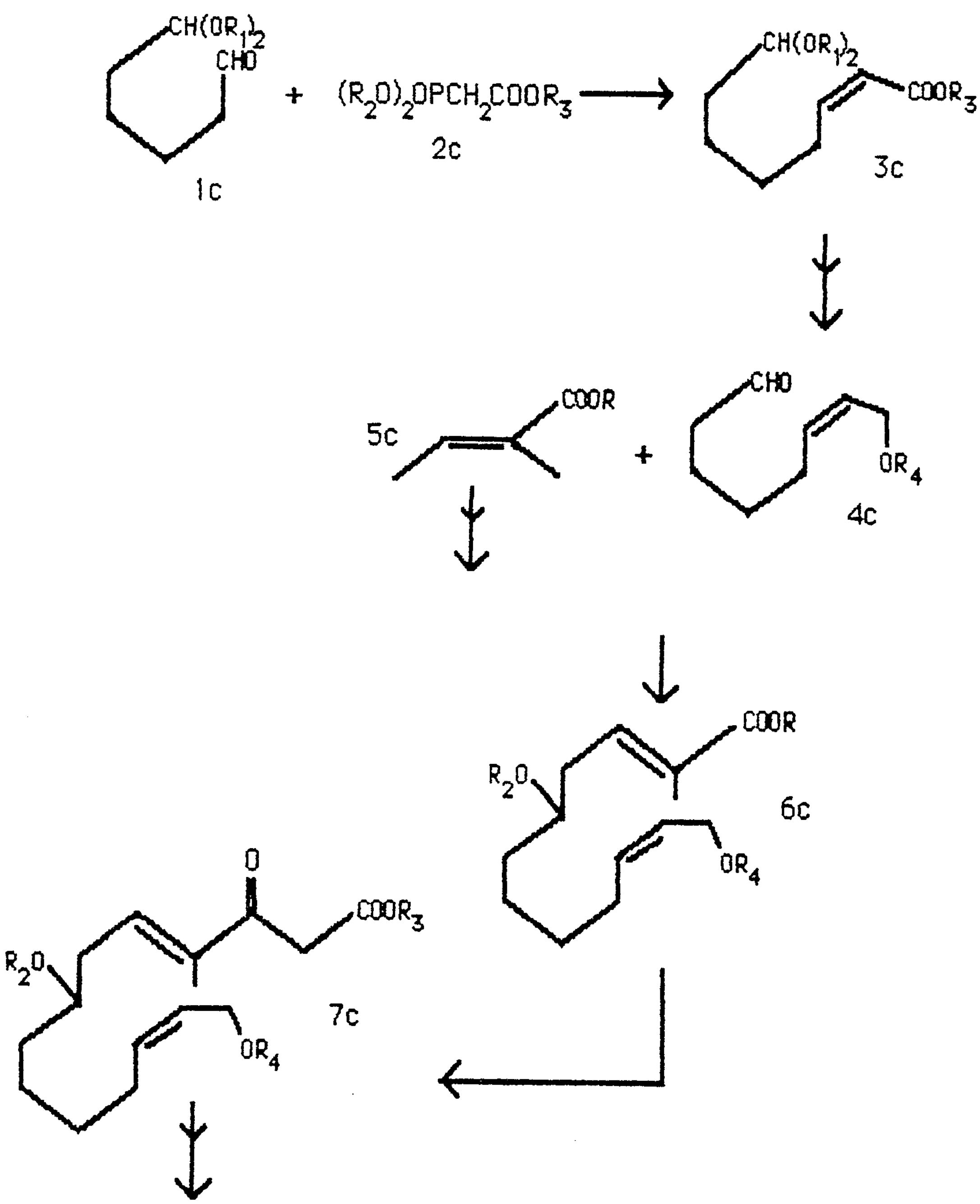


1c

2c

3c

4c

5c

6c

7c

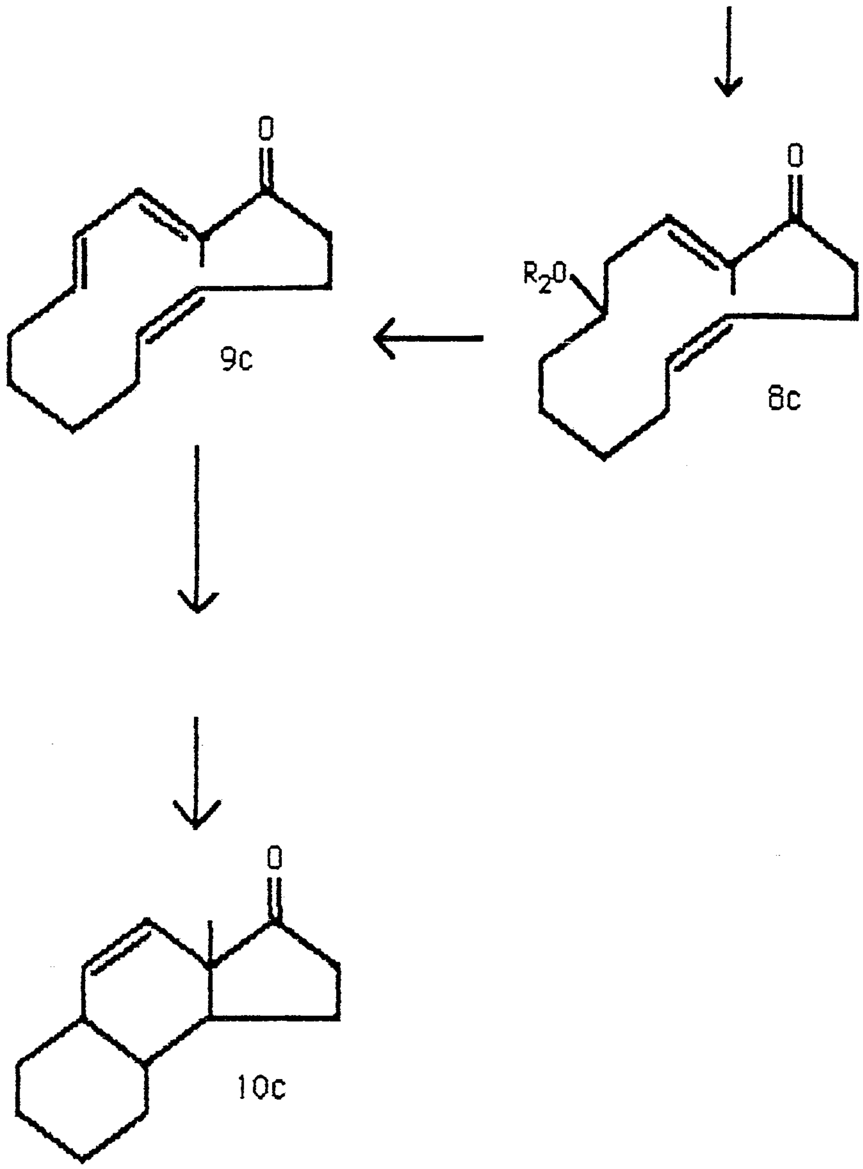

8c

9c

10c

The synthetic process will be more readily illustrated by the following examples which do not intend to limit the present invention thereto.

**Example 1:**Preparation of 5,5,12,12-tetrakis (methoxycarbonyl)-1-methyl-*cis*-1,10-*cisoid*-1,2-*trans*-2-tricyclo [8.4.0.0$^{2,7}$] tetradec-8-ene (tricyclic compound CST 8) (see flow sheet 1).

a) Preparation of (Z) methyl 8-*tert*-butyldimethylsilyloxy-2-methoxycarbonyl-5-methyl-5-octenoate(malonate 2)

To a cooled solution (0ºC) containing (Z)-6-tert-butyldimethylsilyloxy-3-hexen-1-ol(alcohol 1)(4,41 g, 18 mmol) and triethylamine (2,74 g, 27 mmol) in 125 mL of dichloromethane was added dropwise methansulfonyl chloride (2.17 g, 19 mmol). The mixture was stirred at 0º C for 15 minutes and then poured on 200 mL an ice-water mixture. The resulting mixture was extracted in three 150 mL portions of dichloromethane and the combined organic layers were washed with 100 mL of $NH_4Cl$(sat.) and dried over $MgSO_4$. Evaporation of the solvent gave 6 g of the crude mesylate used without further purification for the next reaction.

To a cooled suspension (0ºC) of NaH (1.9 g, 81 mmol) in 200 mL of a 1 to 1 THF:DMF mixture was added slowly dimethyl malonate (11.9 g, 90 mmol). The mixture was warmed to room temperature and crude mesylate (18 mmol) along with KI (250 mg) dissolved in 100 mL of a 1:1 THF:DMF mixture were added. The mixture was heated to 80ºC for 12 h. After cooling to room temperature, the reaction mixture was poured over 400 mL of $NH_4Cl$(sat) and extracted with a 2:1 ether:hexane mixture (3 x 250 mL). The combined organic layers were washed with water (2 x 100 mL) and dried over $MgSO_4$. Concentration gave crude oil which was subjected to column chromatography (hexane:EtOAc, 90:10) to give (Z) methyl 8-*tert*- butyldimethylsilyoxy-2-methoxycarbonyl-5-methyl-5-octenoate (malonate 2) (5.2 g, 81% overall).
IR ($CHCl_3$): 2955, 2930, 2860 and 1730 $cm^{-1}$.
NMR ($CDCl_3$) ∂: 5.18 (1H, t, J = 7 Hz, =C-H), 3.74 (6H, s, -$CO_2Me$), 3.56 (2H, t, J = 7 Hz, =$CH_2$-O), 3.33 (1H, t, J = 7 Hz, $CH(CO_2Me)_2$), 2.16 (2H, qua, J = 7 Hz, $CH_2$-$CH_2$-O), 2.03 (4H, m, -$CH_2$-$CH_2$-$CH(CO_2Me)_2$), 1.69 (3H, s, -$CH_3$), 0.88(9H, s, tBuSi), 0.04 (6H, s, $CH_3$ff-Si).

b) Preparation of (2E, 4Z, 10Z)-13-*tert*-butyldimethylsilyloxy-1-*tert*-butyldiphenylsilyloxy-7,7 -bis(methoxycarbonyl)-10-methyl-2,4,10- tridecatriene(triene 3).

To an ice cooled suspension of NaH (45 mg, 1.9 mmol) in THF (10 mL) was added (Z) methyl 8-*tert*-butyldimethylsilyloxy-2-methoxycarbonyl-5-methyl-5-octenoate (malonate 2) (644 mg, 1.8 mmol). The mixture was warmed to room temperature (1 h) and heated to 35-40ºC (1 h). After cooling to room temperature, (2E,4Z)-6-chloro - 1 - *tert* - butyldiphenylsilyloxy-2,4-hexadiene (chloride 2A)(825 mg, 2.2 mmol) dissolved in DMF (20 mL) was added and the mixture stirred for 15 h. After quenching with $NH_4Cl$ (sat) (100 mL), the resulting mixture was extracted with a 2:1 ether:hexane mixture (4 x 100 mL). The combined organic layers were washed with water (25 mL), bicarbonate (25 mL) and brine (25 mL), dried and concentrated to give an oily residue which was purified by column chromatography (hexane:EtOAc, 95:5) to afford (2E, 4Z, 10Z)-13-*tert*-butyldimethylsilyloxy - 1 - *tert* - butyldiphenylsilyloxy-7,7-bis-(methoxycarbonyl)-10-methyl-2,4,10-tridecatriene(triene 3) (1.09 g. 88%).
IR ($CHCl_3$): 2955, 2930, 2860 and 1730$cm^{-1}$
NMR ($CDCl_3$) ∂ : 7.68 and 7.40 (10H, 2 m, ø-Si), 6.60, 6.13, 5.80 and 5.26 (4H, 4 m, C=C-H diene), 5.13 (1H, t, J = 7 Hz, =C-H dienophile), 4.26 (2H, d, J = 4 Hz, =C-$CH_2$-O), 3.70 (6H, s, -$CO_2Me$), 3.54 (2H, t, J = 7 Hz, -$CH_2$-$CH_2$-O), 2,81 (2H, d, J = 8 Hz, $E_2C$-$CH_2C$=), 2.13 (2H, qua, J = 7 Hz, =CH-$CH_2$-$CH_2$-O), 1.90 (4H, s, $E_2C$-$CH_2$-$CH_2$), 1.66 (3H, d, J = 1 Hz, -$CH_3$), 1.07 (9H, s, t-BuSi-ø), 0.88 (9H, s, t-BuSi-$CH_3$), 0.03 (6H, s, Si-$CH_3$).

c) Preparation of (2E,4Z,10Z)-1-*tert*-butyldiphenylsilyloxy-7,7-bis-(methoxycarbonyl)-10-methyl-2,4,10-tridecatriene-13-ol (alcohol 4).

A solution containing (2E, 4Z, 10Z)-13-*tert*-butyldimethylsilyloxy - 1 - *tert* - butyldiphenylsilyloxy - 7,7 - bis-(methoxycarbonyl)-10-methyl-2,4,10- tridecatriene(triene 3)(105 g, 1.5 mmol) and pyridinium tosylate (38 mg, 0.15 mmol) in isopropyl alcohol (20 mL) was stirred at 70ºC for 17 h. After cooling to room temperature, sodium carbonate was added (50 mg) and the solvent evaporated. The residue was purified by column chromatography (hexane:AcOEt, 75:25) affording (2E, 4Z, 10Z) - 1 - *tert*-butyldiphenylsilyloxy-7,7 - bis - (methoxycarbonyl)-10-methyl-2,4,10-tridecatriene-13-ol(alcohol 4)(790 mg, 90%.
IR ($CHCl_3$): 3000, 2950, 2930, 2860 and 1730 $cm^{-1}$
NMR ($CDCl_3$) ∂: 7.66 and 7.40 (10H, 2 m, ø-Si), 6.62, 6.13, 5.80 and 5.25 (4H, 4m, C=C-H diene), 5.14 (1H, t, J = 7 Hz, C=C-H dienophile), 4.27 (2H, d, J = 4 Hz, =CH-$CH_2$-O), 3.72 (6H, s, $CO_2Me$), 3.57 (2H, t, J = 7 Hz, $CH_2$-$CH_2$-O), 2.83 (2H, d, J = 8 Hz, $E_2C$-$CH_2$-C=), 2.18 (2H, qua, J = 7 Hz, =CH-$CH_2$-$CH_2$-O), 1.92 (4H, s, $E_2C$-$CH_2$-$CH_2$-C=), 1.70 (3H, d, J = 1 Hz, -$CH_3$), 1.62 (1H, s, -OH), (9H, t-BuSi).

d) Preparation of (5Z, 11Z, 13E) methyl 15 - *tert*-butyldiphenylsilyloxy -2, 9, 9 - tris (methoxycarbonyl)-6-methyl-5, 11, 13- pentadecatrienoate(tetraester 5).

To an ice-cooled solution of (2E, 4Z, 10Z)-1-*tert*-butyldiphenylsilyloxy-7,7-bis-(methoxycarbonyl)-10-methyl-2,4,10- tridecatriene- 13-ol(alcohol 4) (126 mg, 0.22 mmol) and triethylamine (33 mg, 0.33 mmol) in dichloromethane (5 ml) was added dropwise methanesulfonyl chloride (34 mg, 0.29 mmol) over 5 min. The mixture was stirred at 0ºC for 15 min and then poured over 20 mL of ice-water and extracted with dichloromethane (3 x 15 mL). The combined organic layers were washed with water (3 x 10 mL), dried over $MgSO_4$ and concentrated to give crude mesylate which was used without further purification.

To an ice-cooled suspension of NaH (25 mg, 1.0 mmol) in 2 mL of DMF was added dimethylmalonate (145 mg, 1.1 mmol) dropwise. The mixture was warmed up to room temperature and the crude mesylate (0.22 mmol) was added dissolved in 4 mL of a 3 to 1 mixture of DMF and THF along with KI (4 mg). The resulting mixture was heated to 80ºC and stirred at this temperature for 12 h then cooled to room temperature, quenched with water (20 mL) and extracted with a 2 to 1 ether:hexane mixture (3 x 15 mL). The combined organic layers were washed with water (2 x 5 mL), dried over $MgSO_4$ and concentrated to give an oily residue which was treated by column chromatography (hexane:acetone, 80:20) to afford (5Z,11Z,13E) methyl 15 - *tert* - butyldiphenylsilyloxy - 2, 9, 9 - tris(methoxycarbonyl)-6-methyl-5,11,13-pentadecatrienoate(tetraester 5) (128 mg. 85% overall).
IR ($CHCl_3$): 3020, 2955, 2930, 2860 and 1730 $cm^{-1}$.
RMN ($CDCl_3$) ∂ : 7.66 and 7.40 (10H, 2 m, ø-Si), 6.62, 6.13, 5.80 and 65.25 (4H, 4 m, C=C-H diene), 5.07 (1H, m, C=C-H dienophile), 4.27 (2H, d, J = 4 Hz, -$CH_2$-O), 3.71 and 3.70 (12H, 2 s, -$CO_2Me$), 3.34 (1H, m, $CH(CO_2Me)_2$), 2.81 (2H, d, J = 8 Hz, $E_2C$-$CH_2$-C=), 1.90 (6H, m, $E_2C$-$CH_2$-$CH_2$-C=C-$CH_2$-), 1.65 (3H, d, J = 1 Hz, -$CH_3$), 1.07 (9H, s, t-BuSi).

e) Preparation of (5Z, 11Z, 13E) methyl 15-hydroxy-2,9,9-tris(methoxycarbonyl)-6-methyl-5,11,13-pentadecatrienoate (alcohol 6)

To a solution containing (5Z, 11Z, 13E) methyl 15 - *tert*-butyldiphenylsilyloxy- 2,9,9 - tris(methoxycarbonyl)-6-methyl-5,11,13- pentadecatrienoate(tetraester 5) (110 mg, 0.16 mmol) in THF (3 mL) was added tetrabutylammonium fluoride (0.32 mmol). The mixture was stirred for 3 h then concentrated under vacuum to give a residue which was purified by column chromatography (hexane:EtOAc, 2:1) to afford (5Z,11Z,13E) methyl 15 - hydroxy-2,9,9- tris (methoxycarbonyl) - 6 - methyl - 5, 11, 13 - pentadecatrienoate (alcohol 6) (51 mg, 71%).
IR ($CHCl_3$): 3020, 2955 and 1730 $cm^{-1}$.
NMR ($CDCl_3$) ∂ : 6.60, 6.14, 5.88 and 5.26 (4H, 4m, C=C-H diene), 5.16 (1H, m, C=C-H dienophile), 4.23 (2H, d, J= 5Hz, $-CH_2-O$), 3.73 and 3.72 (12H, 2 s, $-CO_2Me$), 3.34 (1H, t, J = 7 Hz, $CH(CO_2ME)_2$), 2.83 (2H, dd, J = 1 Hz and 8 Hz, $E_2C-CH_2-C=$), 1.90 (8H, m, $CH_2-CH_2-$), 1.65 (3H, d, J = 1 Hz, $-CH_3$).

f) Preparation of (3E, 5Z, 11Z)-1,1,8,8-tetrakis(methoxycarbonyl)-11-methyl- 3,5,11-cyclotetradecatriene(macrocycle TCC 7).

To an ice-cooled solution of (5Z, 11Z, 13E) methyl 15-hydroxy-2,9,9- tris (methoxycarbonyl) - 6 - methyl - 5, 11, 13-pentadecatrienoate (alcohol 6) (16 mg, 0.035 mmol), LiCl (9.5 mg, 0.22 mmol) and s-collidine (12.8 mg, 0.11 mmol) in DMF (1 mL) was added dropwise methanesulfonyl chloride (12 mg, 0.11 mmol). The mixture was stirred at room temperature for 12 h and then poured over ice-water (10 mL) and extracted with a 2 to 1 mixture of ether:hexane (3 x 10 mL). The combined organic layers were washed with water (5 mL), $Cu(NO_3)_2$(sat)(5 mL) and dried over $MgSO_4$ to afford an oil (15 mg) used without further purification.

A stirred solution containing cesium carbonate (52 mg, 0.16 mmol) and the crude chloride (0.032 mmol) in a 1 to 1 mixture of THF:DMF (6.4 mL) was heated to 80ºC for 4 h. After cooling to room temperature, the carbonate was filtered off and the residue purified by column chromatography(hexane:EtOAc, 10:1/3:1) to afford (3E,5Z,11Z)- 1,1,8,8,- tetrakis (methoxycarbonyl) - 11 - methyl - 3,5, 11 - cyclotetradecatriene (macrocycle TCC 7) (9.9 mg, 64% overall). Similar treatment of the purified chloride afforded (3E,5Z,11Z)-1,1,8,8-tetrakis (methoxycarbonyl) - 11 - methyl - 3, 5, 11 -cyclotetradecatriene(macrocycle TCC 7), in 81% cyclization yield (using slow addition technique).
IR ($CHCl_3$): 3020, 2955, 1730 $cm^{-1}$.
NMR ($CDCl_3$) ∂: 6.56, 6.16, 5.43 and 4.99 (4H,4m, C=C-H diene), 5.14 (1H, m, C=C-H dienophile) 3.74 and 3.73 (12H, 2 s, $-CO_2Me$), 2.80 (4H, m, $E_2C-CH_2-C=$), 1.75 (8H, m, $-CH_2-CH_2-$), 1.65 (3H, d, J = 1 Hz, $-CH_3$).

g) Preparation of 5,5,12,12-tetrakis(methoxycarbonyl)-1-methyl*cis*-1,10-*cisoid*-1,2-*trans*-2-tricyclo [8.4.0.0$^{2,7}$] tetradec-8-ene (tricyclic compound CST 8).

A solution containing (3E,5Z,11Z)- 1,1,8,8- tetrakis-(methoxycarbonyl)- 11-methyl-3,5,11- cyclotetradecatriene (macrocycle TCC 7) (3.6 mg) in tolulene (1.8 mL) was heated at 300ºC in a sealed tube for 1.75 h. Evaporation of toluene gave quantitatively 5,5,12,12-tetrakis- (methoxycarbonyl)-1-methyl-*cis*-1,10-*cisoid*-1,2-*trans*-2-tricyclo [8.4.0.0$^{2,7}$] tetradec-8-ene (tricyclic compound CST 8).
NMR ($CDCl_3$) ∂: 5.32 (2H, s, CH=CH), 3.76, 3.69, 3.68 and 3.66 (12H, 4 s, $-CO_2Me$), 1.02 (3H, s, $-CH_3$).

The present example will be more readily understood by referring to the following flow sheet 1.

## Flow sheet 1

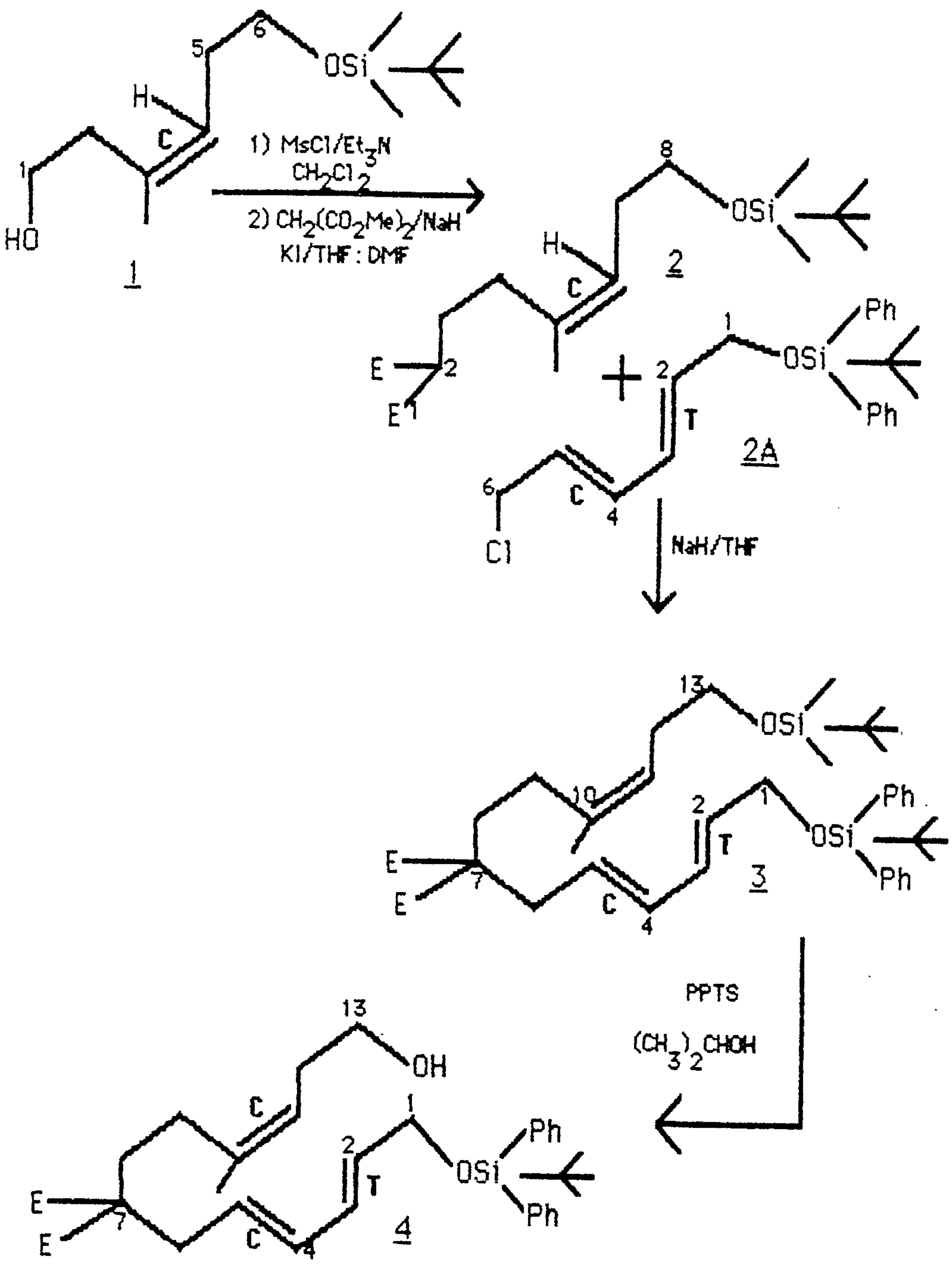


1

2

2A

3

4

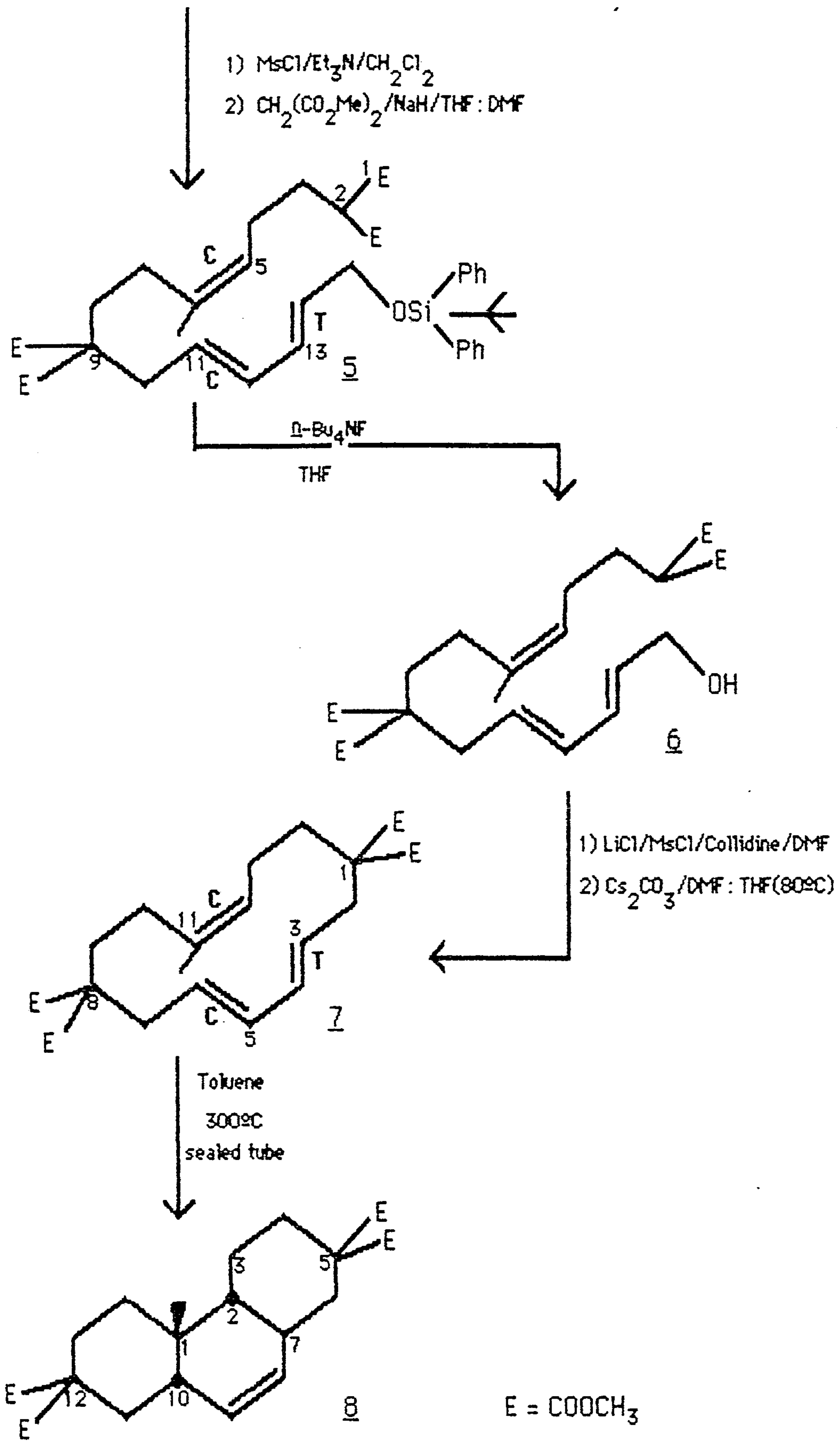


5

6

7

8

E = $COOCH_3$

**EXAMPLE 2:** Preparation of 5,5,12,12-tetrakis-(methoxycarbonyl)-1-methyl-*cis*-1,10-*transoid*-1,2-*cis*-2-tricyclo [8.4.0.0$^{2,7}$]tetradec-8-ene(tricyclic compound CAC 16) (see flow sheet 2).

a) Preparation of (E)methyl 2,9-bis(methoxycarbonyl)-5-methyl)-5- nonenoate(malonate 10)

To an ice-cooled solution containing (E)methyl 8-hydroxy-6-methyl-5-octenoate(alcohol 9) (425 mg, 2.3 mmol) and triethylamine (345 mg, 3.4 mmol) in dichloromethane (20 mL) was added dropwise methanesulfonyl chloride (274 mg, 2.4 mmol). The mixture was stirred for 30 min. at 0ºC, poured over ice-water (25 mL) and extracted with dichloromethane (3 x 25 mL). The combined organic layers were washed successively with water (20 mL), $NH_4Cl$ (sat) (20 mL) and water (40 mL) and then dried over $MgSO_4$. Concentration gave 610 mg of a crude oil used directly for the next reaction.

To an ice-cooled suspension of NaH (246 mg. 10.3 mmol) in 20 mL of a 1 to 1 mixture of THF:DMF was added dropwise dimethyl malonate (150 g, 11.4 mmol). The mixture was then warmed up to room temperature and crude mesylate (2.3 mmol) along with KI (38 mg) dissolved in a 1 to 1 mixture of DMF:THF (10 mL) were added. The mixture was heated to 80-90ºC for 12 h and then poured on $NH_4Cl$ (sat) (50 mL) after cooling to room temperature. The resulting mixture was extracted with a 2 to 1 ether:hexane mixture (3 x 75 mL) and the combined organic layers were washed with water (3 x 25 mL) and dried over $MgSO_4$. The residue after evaporation was treated by column chromatography (hexane:EtOAc, 80:20) affording (E) methyl 2,9-bis(methoxycarbonyl)-5-methyl-5-nonenoate(malonate 10). (540 mg, 79% overall).

IR ($CHCl_3$): 3020, 2955 and 1730 $cm^{-1}$.

NMR ($CDCl_3$)$\partial$: 5.09 (1H, t, J = 7 Hz, =C-H), 3.72 (6H, s, (-$CO_2Me$)$_2$), 3.65 (3H, s, -$CO_2Me$), 3.33 (1H, m, -CH($CO_2Me$)$_2$), 2.29 (2H, t, J = 7 Hz, -$CH_2$-$CO_2Me$), 2.00 (6H, m, $E_2C$-$CH_2$-$CH_2$-C=C-$CH_2$-), 1.65 (2H, qui, J = 7 Hz, -$CH_2CH_2CO_2Me$), 1.57 (1.57(3H, d, J = 1 Hz, -$CH_3$)).

b) Preparation of (5E, 11Z, 13E) methyl 15 - *tert* - butyldiphenylsilyloxy-9,9-bis(methoxycarbonyl)-6-methyl-5,11,13-pentadecatrienoate(triester 11)

To an ice-cooled suspension of NaH (46 mg, 1.9 mmol) in 10 mL of a 1 to 1 mixture of DMF:THF was added (E) methyl 2,9-bis-(methoxycarbonyl)-5-methyl-5- nonenoate(malonate 10). (520 mg, 1.7 mmol) dissolved in 16 mL of the same mixture. The resulting mixture was stirred at 0º for 30 min and warmed up to room temperature. (2E, 4Z)-6 - chloro - 1 - *tert*- butyldiphenylsilyloxy- 2,4- hexadiene (chloride 2A)(700 mg, 1.9 mmol) dissolved in 24 mL of the above solvent mixture was added and stirring went on for 8 h. The reaction mixture was quenched with $NH_4Cl$ (sat) (10 mL) and 100 mL of water were added followed by extraction with a 2 to 1 ether:hexane mixture (4 x 100 mL). The combined organic layers were washed with water (2 x 75 cc), brine (1 x 75 mL) and dried over $MgSO_4$. Concentration gave a ridue which was purified by column chromatography (hexane:ether, 80:20) to afford (5E, 11Z, 13E) methyl 15-*tert*-butyldiphenyl silyloxy-9,9-bis (methoxycarbonyl)-6-methyl-5,11,13 -pentadecatrienoate(triester 11) (930 mg, 84%).

IR ($CH_2Cl_2$): 2960, 2860, 1730 $cm^{-1}$.

NMR ($CDCl_3$)$\partial$: 7.68 and 7.40 (10H, 2 m, ø-Si), 6.60, 6.13, 5.80 and 5.25 (4H, 4 m, C=C-H diene), 5.10 (1H, m, m, C=C-H dienophile), 4.26 (2H, d, J = 5 Hz, -$CH_2$-O), 3.70 (6H, s, -($CO_2Me$)$_2$), 3.65 (3H, s, -$CO_2Me$), 2.81 (2H, d, J = 8 Hz, $E_2C$-$CH_2$-C=), 2.28 (2H, t, J = 7 Hz, -$CH_2$-$CO_2Me$), 1.90 (6H, m, $E_2C$-$CH_2$-$CH_2$-C=C-$CH_2$-), 1.65 (2H, qui, J = 7 Hz, -$CH_2$-$CH_2$-$CO_2Me$), 1.56 (3H, d, J = 1 Hz, -$CH_3$), 1.07 (9H, s, t-BuSi).

c) Preparation of (5E, 11Z, 13E) methyl 15- *tert* - butyldiphenylsilyloxy - 2,9,9 - tris (methoxycarbonyl) - 6 - methyl -5, 11, 13-pentadecatrienoate(tetraester 12)

To an ice cooled solution of a diisopropylamine (101 mg, 1.0 mmol) in THF was added n-BuLi (0.95 mmol). The mixture was stirred and kept at 0ºC (total volume 2.55 mL).

To a solution containing (5E, 11Z, 13E) methyl 15-*tert*-butyldiphenylsilyloxy - 9,9 - bis (methoxycarbonyl) - 6 - methyl-5,11,13- pentadecatrienoate(triester 11) (165 mg, 0.26 mmol) in 2 mL THF at -78ºC was added 695µL (0.26 mmol) of the LDA solution. After 30 min, methyl chloroformate (12.2 mg, 0.13 mmol) was added and the mixture stirred for 20 min at -78ºC after which 348 µL (0.13 mmol) of LDA was added followed after 20 min by 7.3 mg (0.08 mmol) of methyl chloroformate. The process was repeated two more times cutting amounts of reactants by half in each addition. The final reaction mixture was quenched with $NH_4Cl$ (sat) (5 mL) and extracted with ether (3 x 10 mL). The combined organic layers were washed with water (5 mL) and dried over $MgSO_4$ to afford an oil which was purified by column chromatography (hexane:EtOAc, 90:10/70:30) giving (5E, 11Z, 13E) methyl 15 - *tert* - butyldiphenylsilyloxy -2,9,9 - tris (methoxycarbonyl) - 6 - methyl -5, 11, 13 - pentadecatrienoate (tetraester 12) (126 mg, 70%).

IR ($CHCl_3$): 3020, 2960, 2860 and 1730 $cm^{-1}$.

NMR ($CDCl_3$)$\partial$: 7.68 and 7.40 (10H, 2 m, ø-Si), 6.60, 6.13, 5.80 and 5.25 (4H, 4 m, C=C-H diene), 5.10 (1H, m, C=C-H dienophile), 4.26 (2H, d, J = 5 Hz, -$CH_2$-O), 3.72 and 3.69 (12H, 2s, -$CO_2Me$, 3.35 (1H, t, J = 7 Hz CH($CO_2Me$)$_2$), 2.79 (2H, d, J = 8 Hz, $E_2C$-$CH_2$-C=), 1.90 (8H,m, $CH_2$-$CH_2$-C=), 1.55 (3H, d, J = 1 Hz, -$CH_3$), 1.07 (9H, s, t-BuSi).

d) Preparation of (5E, 11Z, 13E) methyl 15-hydroxy-2,9,9-tris(methoxycarbonyl)-6-methyl-5,11,13-pentadecatrienoate (alcohol 13)

To a solution containing (5E, 11Z, 13E) methyl 15- *tert* - butyldiphenylsilyloxy - 2,9,9 - tris (methoxycarbonyl) - 6 - methyl -5, 11, 13-pentadecatrienoate (tetraester 12) (110 mg, 0.16 mmol) in THF (2.5 mL) was added

tetrabutylammonium fluoride (0.17 mmol). The resulting mixture was stirred for 1 h and the solvent evaporated to give a residue which was purified by column chromatography (hexane: EtOAc, 2:1/1:1) affording (5E,11Z,13E) methyl 15- hydroxy-2,9,9- tris(methoxycarbonyl)-6-methyl-5,11,13-pentadecatrienoate (alcohol 13) (53 mg, 73%).
IR ($CHCl_3$): 3600, 3020, 2960 and 1735 $cm^{-1}$.
NMR ($CDCl_3$)$\partial$: 6.53, 6.12, 5.86 and 5.25 (4H, 4m, C=C-H diene), 5.08 (1H, m, C=C-H dienophile), 4.21 (2H, d, J = 7 Hz, $-CH_2-O$) 3.73 and 3.70 (12H, 2s, $-CO_2Me$), 3.34 (1H, t, J = 7 Hz, $CH(CO_2Me)_2$), 2.80 (2H, dd, J = 1 and 8 Hz, $E_2C-CH_2-C=$), 1.90 (8H, m, $-CH_2-CH_2-$), 1.64 (1H, s, -OH), 1.55 (3H, d, J = 1 Hz, $-CH_3$)

e) Preparation of (5E, 11Z, 13E) methyl 15-chloro-2,9,9-tris (methoxycarbonyl) - 6 - methyl-5,11,13-pentadecatrienoate (chloride 14).

To an ice-cooled solution containing (5E, 11Z, 13E) methyl 15 - hydroxy - 2,9,9 - tris(methoxycarbonyl)-6-methyl-5, 11, 13-pentadecatrienoate (alcohol 13) (160 mg, 0.35 mmol), LiCl (59 mg, 1.4 mmol) and s-collidine (68 mg, 0.56 mmol) in DMF (5 mL) was added dropwise methanesulfonyl chloride (60 mg, 0.52 mmol). The mixture was stirred at 25ºC for 3 h then poured over 50 ml of cold water and extracted with a 2:1 mixture of ether:hexane (3 x 40 mL). The combined organic layers were washed with water (230 mL), $Cu(NO_3)_2$ (20 mL) and again water (20 mL). After drying with $MgSO_4$ and concentration, the residue was purified by column chromatography (hexane:EtOAc, 90:10) to afford (5E, 11Z, 13E) methyl 15- chloro-2,9,9- tris(methoxycarbonyl)-6-methyl-5,11,13-pentadecatrienoate (chloride 14). (147 mg, 89%).
IR ($CHCl_3$): 3010, 2950 and 1730 $cm^{-1}$.
NMR ($CDCl_3$)$\partial$: 6.56, 6.11, 5.83 and 5.35 (4H, 4 m, C=C-H diene), 5.08 (1H, m, C=C-H dienophile), 4.12 (2H, dd, J = 1 and 7 Hz, $-CH_2-Cl$), 3.73 and 3.71 (12H, 2 s, $-CO_2Me$), 3.35 (1H, t, J = 7 Hz, $CH(CO_2Me)_2$), 2.81 (2H, dd, J = 1 and 8 Hz, $E_2C-CH_2-C=$), 1.90 (8H, m, $CH_2-CH_2$), 1.55 (3H, d, J = 1 Hz, $-CH_3$).

f) Preparation of 3E,5Z,11E) - 1,1,8,8 -tetrakis (methoxycarbonyl)-11-methyl-cyclotetradeca-3,5,11-triene (macrocycle TCT 15).

To a suspension of cesium carbonate (103 mg, 0.32 mmol) in a 1 to 1 mixture of THF:DMF (8 mL) at 85ºC was added dropwise over 5 h a solution containing (5E, 11Z, 13E) methyl 15- chloro - 2,9,9 -tris (methoxycarbonyl) - 6 - methyl - 5, 11, 13- pentadecatrienoate (chloride 14) (30 mg, 0.06 mmol) in 9 mL of a 5 to 4 DMF:THF mixture. After the addition was completed, the mixture was stirred an additional 1 h and then cooled to room temperature. The carbonate was filtered off and solvents distilled off under vacuum to leave an oily residue which was purified by column chromatography (hexane:EtOAc, 90:10) affording (3E,5Z,11E)-1,1,8,8- tetrakis (methoxycarbonyl)-11-methyl-cyclotetradeca-3,5,11-triene (macrocycle TCT 15). (24 mg, 87%).
IR ($CHCl_3$): 3020, 2955 and 1730 $cm^{-1}$.
NMR ($CDCl_3$)$\partial$: 6.13, 5.25, and 5.01 (5H, 3 m, C=C-H ), 3.74 and 3.73 (12H, 2 s, $-CO_2Me$), 2.76 (4H, t, J = 7 Hz, $E_2C-CH_2-C=$), 1.90 (8H, m, $CH_2-CH_2-$), 1.58 (3H, d, J = 1 Hz, $-CH_3$).

g) Preparation of 5,5,12,12-tatrakis (methoxycarbonyl)-1-methyl - *cis*-1, 10- *transoid*-1,2-*cis*-2-tricyclo[8.4.0.0$^{2,7}$] tetradec-8-ene(tricyclic compound CAC 16)

(3E,5Z,11E)-1,1,8,8- tetrakis (methoxycarbonyl)-11-methylcyclotetradeca-3,5,11-triene (macrocycle TCT 15). (5.5 mg) was heated neat in a sealed tube at 300ºC for 2 h affording 5,5,12,12-tetrakis (methoxycarbonyl) - 1 - methyl - *cis*-1, 10- *transoid*-1,2 - *cis* -2-tricyclo[8.4.0.0$^{2,7}$]tetradec-8-ene(tricyclic compound CAC 16) (5 mg, 90%).
NMR ($CDCl_3$)$\partial$: 5.26 (2H, s, HC=CH), 3.68, 3.66 and 3.65 (12H, 3 s, $-CO_2Me$), 1.00 (3H, s, $-CH_3$).

The present example will be more readily understood by referring to the following flow sheet 2.

## Flow sheet 2

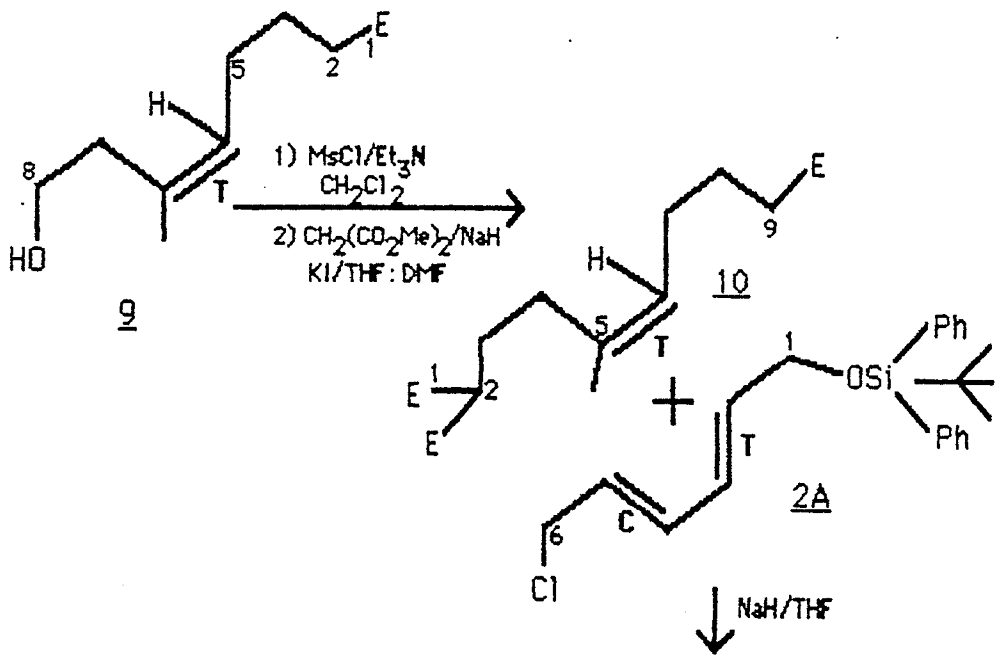


9

10

2A

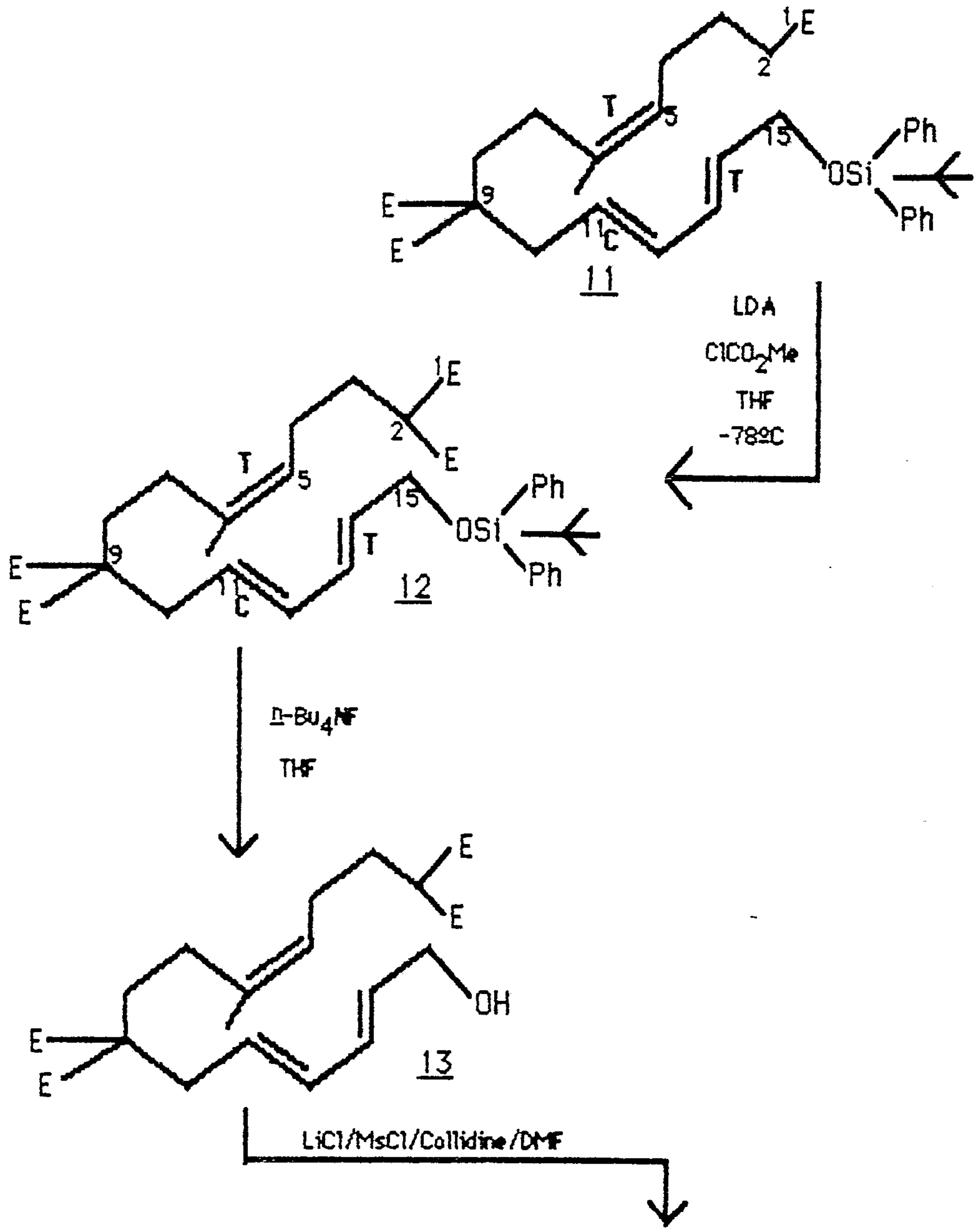
LDA
$ClCO_2Me$
THF
-78ºC
$\underline{n}$-$Bu_4NF$
THF
LiCl/MsCl/Collidine/DMF

11

12

13

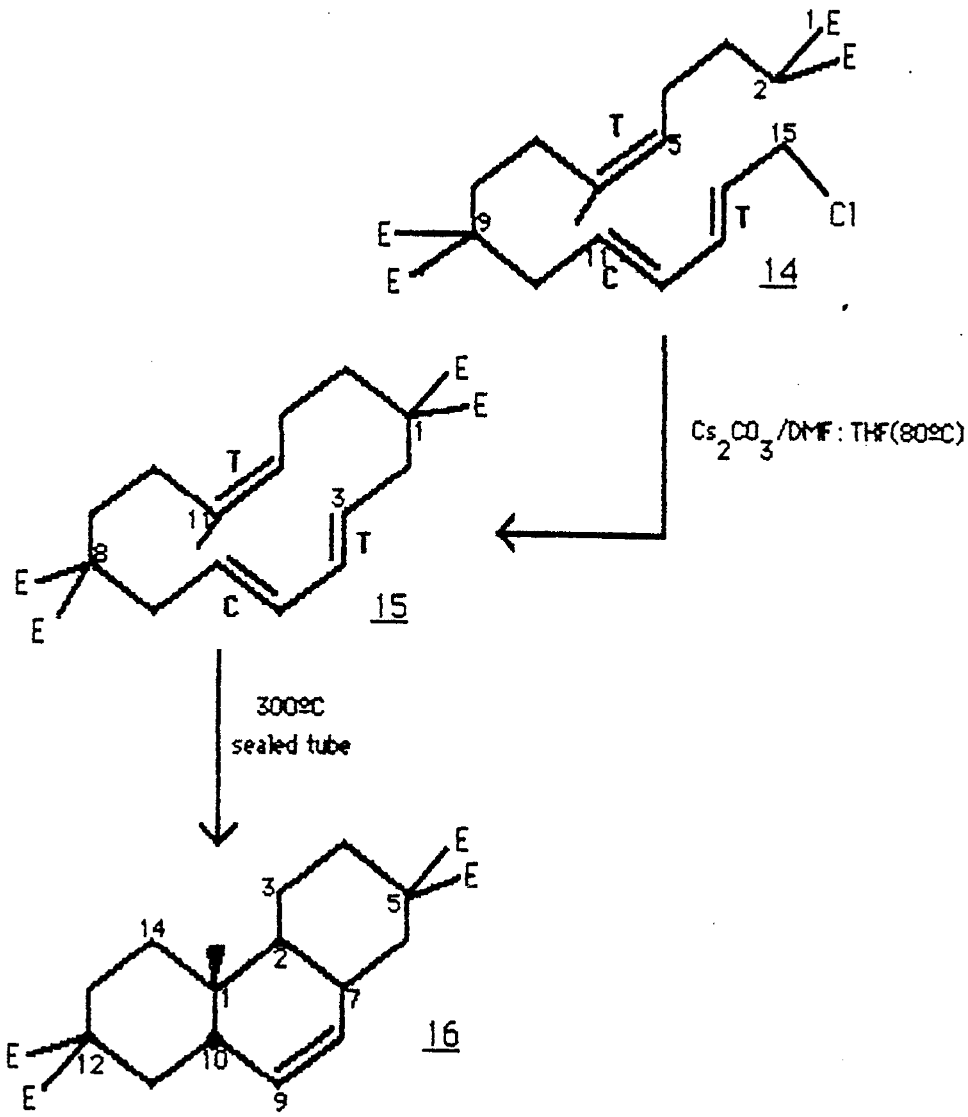


14

15

16

E=COOCH3

**EXAMPLE 3:** Preparation of 5,5,12,12-tetrakis (methoxycarbonyl)- 1-methyl-*trans*-1,10-*cisoid*-1,2-*trans* -2-tricyclo[8.4.0.0$^{2,7}$] tetradec-8-ene(tricyclic compound TST 18)

Following the procedure described in example 1, 5,5,12,12- tetrakis (melthoxycarbonyl)- 1-methyl-*trans*-1,10-*cisoid*-1,2-*trans*-2-tricyclo[8.4.0.0$^{2,7}$] tetradec-8-ene(tricyclic compound TST 18) was prepared from compound 17 and analysed.

NMR ($CDCl_3$)∂: 5.61 (2H, m, HC=CH), 3.75, 3.74, 3.70 and 3.69 (12H, 4 s, -$CO_2$Me), 0.80 (3H, s, $CH_3$).

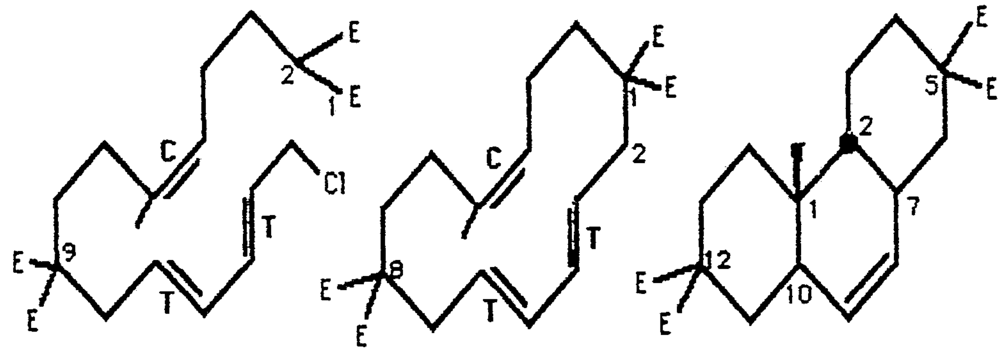

CTT 17 (TTC not isolated) TST 18

E=$COOCH_3$

**Example 4:** Preparation of 5, 5, 12, 12 - tetrakis (methoxycarbonyl) - 1 - methyl - *cis* - 1, 10 - *transoid*-1, 2- *cis* -2-tricyclo [8.4.0.0$^{2,7}$]tetradec-8-ene (tricyclic compound CAC 21).

Following the procedure described in example 2, (3Z,5E,11E)- 1,1,8,8 - tetrakis (melthoxycarbonyl) - 11 - methyl -cyclotetradeca - 3,5,11-triene(macrocycle CTT 20) was prepared from compound 19 and analysed.

NMR ($CDCl_3$)$\partial$: 6.25-4.89 (5H, m, olefins), 3.75, 3.74, 3.73 and 3.72 (12H, 4 s, $-CO_2Me$), 2.79 (4H, m, $E_2C-CH_2-C=$), 2.02-1.78 (8H, m, $-CH_2-CH_2-$), 1.59 (3H, s, $-CH_3$).

Diels-Alder reaction of this macrocyclic triene afforded 5,5,12,12-tetrakis(methoxycarbonyl)-1-methyl- *cis* -1, 10 - *transoid* - 1,2-*cis*-2-tricyclo[8.4.0.0$^{2,7}$]tetradec-8-ene (tricyclic compound CAC 21).

NMR ($CDCl_3$)$\partial$: 5.26 (2H, m, HC=CH), 3.68, 3.66 and 3.65 (12H, 3 s, $-CO_2Me$), 1.00 (3H, s, $-CH_3$).

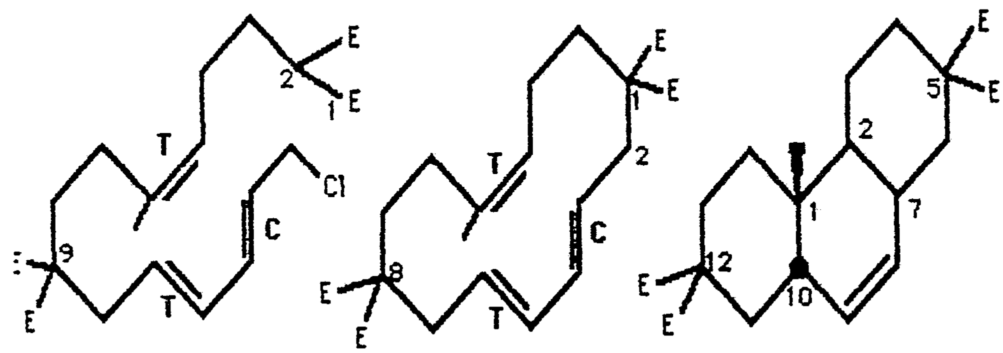

TTC 19 CTT 20 CAC 21

E=$COOCH_3$

**Example 5:** Preparation of 5,5,12,12-tetrakis(methoxycarbonyl) - 1 - methyl - *trans*-1, 10- *cisoid*-1,2-*cis*-2-tricyclo [8.4.0.0$^{2,7}$]tetradec-8-ene(tricyclic compound TSC 24).

Following the procedure described in example 1, (3Z,5E,11Z)- 1,1,8,8 - tetrakis (methoxycarbonyl) - 11 - methylcyclotetradeca- 3,5,11-triene(macrocycle CTC 23) was prepared from compound 22 and analysed.

NMR ($CDCl_3$)$\partial$: 6.58, 6.15, 5.39 and 5.00 (4H, 4m, =CH diene), 5.06 (1H, m, C=C-H dienophile), 3.75 and 3.73 (12H, 2 s, $-CO_2Me$), 2.81 (4H, m, $E_2C-CH_2-C=$), 1.73 (8H, m, $-CH_2-CH_2-$), 1.65(3H, d, J = 1 Hz, $-CH_3$).

MS (m/e): 436 ($M^+$), 421 ($M-CH_3$), 418 ($M-H_2O$), 404 (M-MeOH).

Diels-Alder reaction of (3Z, 5E, 11Z) - 1,1,8,8 -tetrakis (methoxycarbonyl) - 11 - methyl - cyclotetradeca-3,5,11-triene(macrocycle CTC 23) afforded tricyclic compound 5,5,12,12-tetrakis(methoxycarbonyl)-1-methyl-*trans*-1, 10- *cisoid* -1,2-*cis*-2-tricyclo[8.4.0.0$^{2,7}$]tetradec-8-ene (tricyclic compund TSC 24).

NMR ($CDCl_3$)$\partial$: 5.33, 5.23 (2H, m, HC=CH), 3.76, 3.69, 3.68 and 3.67 (12H, 4s, $-CO_2Me$), 2.71-1.02 (other protons), 0.87 (3H, s, $-CH_3$).

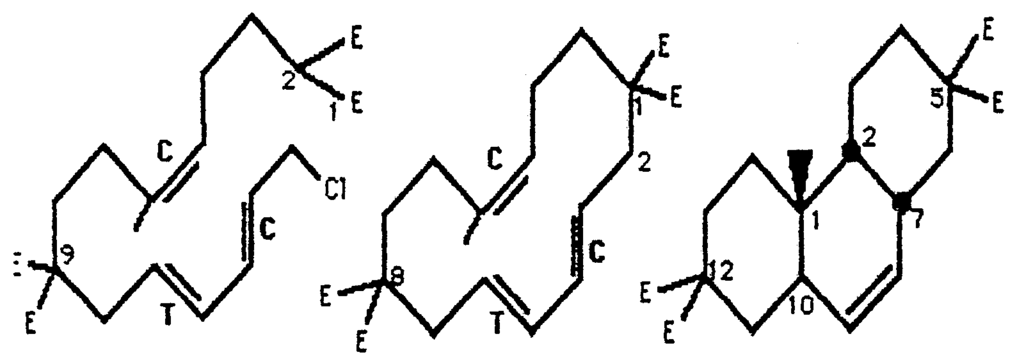

CTC 22 CTC 23 TSC 24

$E=COOCH_3$

**Example 6:** Preparation of 5,5,12,12-tetrakis(methoxycarbonyl)- 1 - methyl - *cis*- 1, 10- *cisoid*-1,2-*cis*-2-tricyclo [8.4.0.0$^{2,7}$]tetradec-8-ene(tricyclic compound CSC 27).

Following the procedure described in example 1, (3Z,5Z,11Z)-1,1,8,8 - tetrakis (methoxycarbonyl)-11 - methylcyclotetradeca- 3,5,11-triene(macrocycle CCC 26) was prepared from compound 25 and analysed.

NMR ($CDCl_3$)$\partial$: 6.45 and 5.40 (4H, 2 m, C=C-H diene), 5.13 (3H, m, C=C-H dienophile), 3.75, 3.73 (12H, 2 s, $-CO_2Me$), 3.10-2.55 and 2.20-1.60 (12H, 2 m, $-CH_2-$), 1.60 (3H, d, J = 1 Hz, $-CH_3$).

Diels-Alder reaction of (3Z,5Z, 11Z) - 1,1,8,8 -tetrakis(methoxycarbonyl) - 11 - methyl - cyclotetradeca-3,5,11-triene (macrocycle CCC 26) gave tricyclic compound 5,5,12,12-tetrakis(methoxycarbonyl)-1-methyl-*trans*-1, 10- *cisoid*-1,2-*cis* -2-tricyclo[8.4.0.0$^{2,7}$]tetradec-8-ene (tricyclic compund CSC 27).

NMR ($CDCl_3$)$\partial$: 5.32, (2H, s, CH=CH), 3.76, 3.68, and 3.66 (12H, 3 s, $-CO_2Me$), 2.70-1.00 (other protons), 1.02 (3H, s, $-CH_3$).

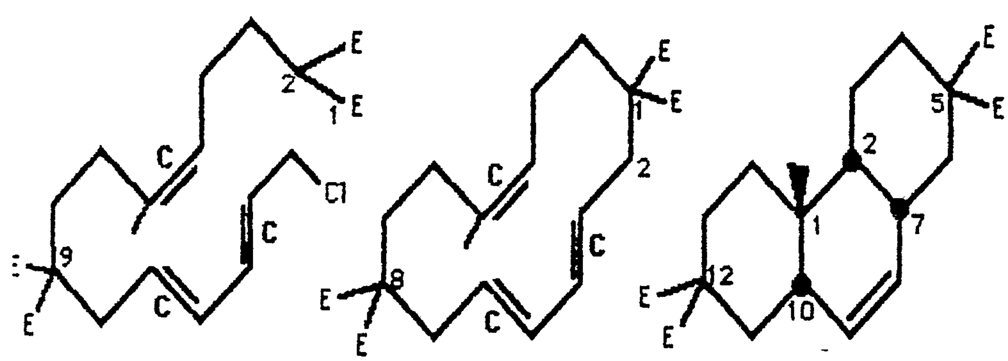

CCC 25 CCC 26 CSC 27

$E=COOCH_3$

**Example 7:** Preparation of 5, 5, 12, 12 - tetrakismethoxycarbonyl)-1,2-dimethyl-*trans*-1,10-*cisoid*-1,2-*trans*-2-tricyclo[8.4.0.0.$^{2,7}$]tetradec-8-ene(tricyclic compound TST 32)

Following the procedure described in example 1, (3E,5E,11Z)-1, 1, 8, 8-tetrakis- (methoxycarbonyl)-11,12-dimethylcyclotetradeca-3,5,11-triene(macrocycle TTC 31) was prepared from compound 30 and analysed.
NMR ($CDCl_3$)∂: 6.19 (2H, m, $H^5$ and $H^4$), 5.74 (2H, m, $H^3$ and $H^6$), 3.73 (12H, s, $OCH_3$), 2.63 (4H, d, J = 7.4 Hz, $CH_2{}^7$ and $CH_2{}^2$), 1.76 (8H, m, $CH_2{}^{9,10,13,14}$), 1.52 (6H, s, $CH_3$).

Diels-Alder reaction of (3E,5E,11Z)-1,1,8,8-tetrakis(methoxycarbonyl)-11,12-dimethyl-cyclotetradeca-3,5,11-triene (macrocycle TTC 31) afforded 5,5,12,12-tetrakis(methoxycarbonyl)-1,2-dimethyl-*trans*-1,10-*cisoid*-1,2-*trans*2-tricyclo[8.4.0.0$^{2,7}$]tetradec-8-ene(tricyclic compound TST 32)
NMR ($CDCl_2$)∂: 5.61 (2H, d, J = 1.5 $H^8$ and $H^9$), 3.75 and 3.70 (12H, 2 s, $OCH_2$), 0.79 (6H, s, $CH_3$).

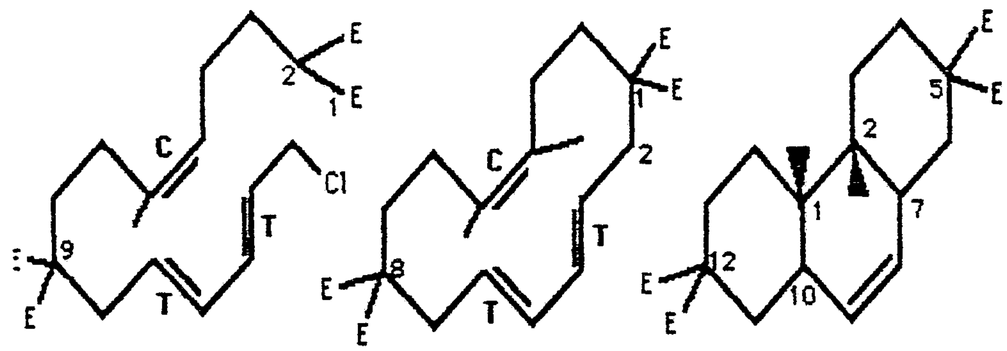

CTT 30 TTC 31 TST 32

E=$COOCH_3$

**Example 8:**Preparation of 5,512,12 - tetrakis(methoxycarbonyl)-1,2-dimethyl-*cis*-1, 10-*cisoid*-1,2-*trans* -2-tricyclo[8.4.0.0$^{2,7}$]tetradec-8-ene (tricyclic compound CST 35)

Following the procedure described in example 1, (3E,5Z,11Z)-1,1,8,8-tetrakis-(methoxycarbonyl)-11,12-dimethylcyclotetradeca-3,5,11-triene (macrocycle TCC 34) was prepared from compound 33 and analysed.
NMR ($CDCl_3$)∂: 6.53 (1H, m, $H^4$), 6.17 (1H, m, $H^5$), 5.43 (1H, m, $H^3$), 5.01 (1H, m, $H^6$), 3.743 and 3.739 (12H, 2 s, $OCH_3$), 2.77 (4H, m, $CH_2{}^7$ and $CH_2{}^{14}$), (1.82 8H, m, $CH_2{}^{9,10,13,2}$), 1.57 (6H, m, $CH_3$).

Diels-ALDer reaction of (3E,5Z,11Z)-1,1,8,8-tetrakis-(methoxycarbonyl) - 11, 12 - dimethyl - cyclotetradeca -3,5,11-triene (macrocycle TCC 34) afforded 5,5,12,12-tetrakis(methoxycarbonyl)-1,2-dimethyl-*cis*-1,10-*cisoid*-1,2-*trans*-2-tricylco[8.4.0.0$^{2,7}$]tetradec-8-ene(tricyclic compound CST 35).
NMR ($CDCl_3$)∂: 5.26 (2H, m, $H^8$ and $H^9$), 3.77, 3.70, 3.69 and 3.66 (12H, 4s, $OCH_3$), 0.93 and 0.80 (6H, 2 s, $CH_3$).

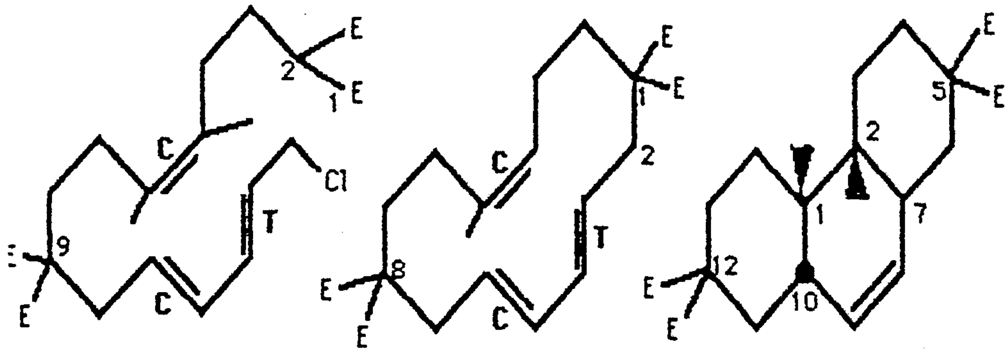

CCT 33 TCC 34 CST 35

$E = COOCH_3$

**Claims**

1. A process for preparing a tricyclic compound having the following formula (I):

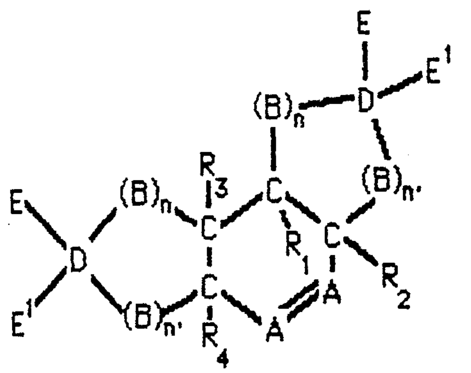

(I)

wherein

$R_1$, $R_2$, $R_3$ and $R_4$ can be the same or different and are hydrogen, lower alkyl, aryl, aldehyde, -SR, -OR or -COOR wherein R is hydrogen, halogen, lower alkyl, or lower aryl; $-CONR_5R_6$ wherein $R_5$ and $R_6$ can be the same or different and can be a hydrogen, lower alkyl or lower aryl; $NR_5R_6$ wherein $R_5$ and $R_6$ are as previously defined;

A can be either nitrogen or a carbon atom, which may be substituted by $R_1$,

B can be either oxygen, sulfur, nitrogen or carbon which may be substituted by $R_1$,

C can be either nitrogen or a carbon atom, which may be substituted by $R_1$,

D can be either oxygen, nitrogen or a carbon atom, which may be substituted by E and $E^1$,

n and n′ can be the same or different and are integers ranging from 0 to 2 and

E and $E^1$ can be the same or different and are hydrogen or a functional group,

which comprises reacting a dienophile having the following general formula XIV:

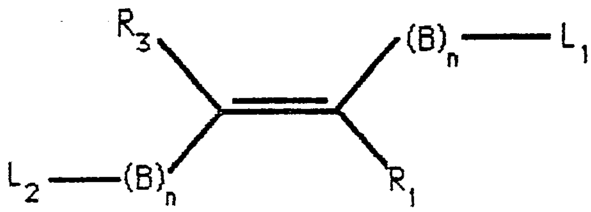

XIV

wherein:

L₁ and L₂ can be the same or different and are suitable leaving groups,

B, n, $R_1$ and $R_3$ are as previously defined,

and a diene XV having the following general formula:

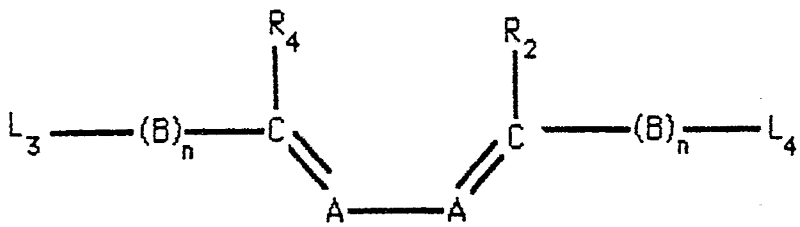

XV

wherein:

$L_3$ and $L_4$ can be the same or different and are suitable leaving groups, and

B, A, C, $R_4$, $R_2$ and n are as previously defined, in the presence of a connector having the following formula:

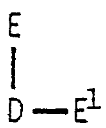

wherein E is oxygen, nitrogen or carbon and E and $E^1$ can be the same or different and are as previously defined, and the macrocyclic triene thus formed subsequently undergoes a transannular Diels-Alder reaction to yield the desired tricyclic compound of formula (I).